# EUROPÄISCHE PATENTSCHRIFT

(11) **EP 2 870 482 B1**
(45) Veröffentlichungstag und Bekanntmachung des Hinweises auf die Patenterteilung: **11.03.2020**
(21) Anmeldenummer: 13750645.7
(22) Anmeldetag: 09.07.2013
(51) Int. Cl.: G01N 35/00, G01N 33/00, G01N 27/04

(54) **VERFAHREN ZUR IDENTIFIZIERUNG UND DISKRIMINIERUNG VON MATERIALIEN FÜR EINEN PRODUKTIONSPROZESS, UND ZUGEHÖRIGES SYSTEM**
METHOD FOR IDENTIFYING AND DISTINGUISHING MATERIALS FOR A PRODUCTION PROCESS, AND CORRESPONDING SYSTEM
PROCÉDÉ D'IDENTIFICATION ET DE DISCRIMINATION DE MATÉRIAUX POUR UN PROCESSUS DE PRODUCTION, ET SYSTÈME CORRESPONDANT

(30) Priorität: 09.07.2012 DE 102012106132; 02.07.2013 DE 102013106915
(43) Veröffentlichungstag der Anmeldung: 13.05.2015
(73) Patentinhaber: Maschinenfabrik Reinhausen GmbH, 93059 Regensburg (DE)
(72) Erfinder: NETTESHEIM, Stefan, 93051 Regensburg (DE)
(74) Vertreter: Reichert & Lindner Partnerschaft Patentanwälte
(86) Internationale Anmeldenummer: PCT/EP2013/064513
(87) Internationale Veröffentlichungsnummer: WO 2014/009384

(56) Entgegenhaltungen:
- EP-B1- 0 606 121
- US-A- 3 526 127
- US-A1- 2006 129 345
- US-A1- 2007 192 032
- US-A1- 2009 289 448
- Anonymous: "Signatur (Programmierung) - Wikipedia", , 18. Dezember 2013 (2013-12-18), XP055094055, Gefunden im Internet: URL:http://de.wikipedia.org/wiki/Signatur_ (Programmierung) [gefunden am 2013-12-18]
- Anonymous: "Digitale Signatur - Wikipedia", , 18. Dezember 2013 (2013-12-18), XP055094057, Gefunden im Internet: URL:http://de.wikipedia.org/wiki/Digitale_ Signatur [gefunden am 2013-12-18]
- Anonymous: "Elektronische Signatur - Wikipedia", , 18. Dezember 2013 (2013-12-18), XP055094058, Gefunden im Internet: URL:http://de.wikipedia.org/wiki/Elektroni sche_Signatur [gefunden am 2013-12-18]
- Anonymous: "Server (computing) - Wikipedia, the free encyclopedia", , 17. Dezember 2013 (2013-12-17), XP055094032, Gefunden im Internet: URL:http://en.wikipedia.org/wiki/Server_(c omputing) [gefunden am 2013-12-17]

## Beschreibung

Die vorliegende Erfindung betrifft ein System zur Identifizierung oder Diskriminierung von Materialien.

Ferner umfasst die Erfindung ein Verfahren zur Identifizierung oder Diskriminierung von Materialien mit einem System.

Um heterogene Stoffe oder Stoffgemische wie z.B. Pulvern, Pasten, Suspensionen, Schüttgütern zu analysieren werden vielfältige Messmethoden eingesetzt, die jeweils auf eine oder mehrere spezifische Eigenschaften abzielen. Pulver können durch Streulichtuntersuchungen bezüglich der Partikelgrößenverteilung vermessen werden. Die chemischen Eigenschaften eines Materials können in hoher Genauigkeit sowohl im Volumen als auch in der Oberflächenzusammensetzung bestimmt werden. Analog können elektrische und magnetische Eigenschaften des Materials oder des Materialgemisches gemessen werden. Der zeitliche und apparative Aufwand dieser Messmethoden ist sehr unterschiedlich. Nicht jede der Methoden ist jedoch geeignet zu einer hinreichend schnellen oder echtzeit-tauglichen Prozesskontrolle.

Pulveranalytische Verfahren und auch genaue Materialanalysen sind meist aufwändige, kostspielige und zeitintensive Verfahren. Besonders bei heterogenen Materialien, wie z.B. Pulvern, Pasten, Suspensionen, Schüttgütern, heterogenen Mischungen, ist die Korrelation zwischen physikalischen und chemischen Eigenschaften und Verhalten im Fertigungsprozess komplex und schwer zu untersuchen. Daher wird meistens auf empirisches Wissen zurückgegriffen. Im Allgemeinen ist es nicht möglich aus kollektiven Eigenschaften einer großen Partikelmenge, wie z.B. eines Schüttgutes oder eines Pulvers, auf die Eigenschaften der einzelnen Partikel zu schließen und vice versa.

Selbst bei genauer chemischer Analyse einer sehr großen Partikelzahl sowie genauster Bestimmung der Partikelgrößenverteilung und der Partikelformen ist es z.B. schwer möglich auf prozessrelevante Größen, wie z.B. die Rieselfähigkeit, die Klumpneigung, die Scheindichte zu schließen. Die Komplexität von Schüttgütern oder Pulvern erschwert die Qualitätssicherung in Fertigungsprozessen.

Bei technisch relevanten Prozessen ist nicht nur die statistische Verteilung der Partikelgrößen und Partikelformen, sondern auch die chemische Zusammensetzung der Partikel im Volumen und an der Oberfläche wichtig. Bei Pulvermischungen ist der Komplexitätsgrad entsprechend höher.

Für eine Produktion, kann es erforderlich sein genau zu erkennen ob sich die Zusammensetzung der zugeführten Ausgangsmaterialien ändert. Jeder Wechsel der Materialcharge, der Materialquelle oder des Lieferanten ist ein Risiko dafür, dass eingefahrene, empirisch beherrschte Prozesse instabil werden. Oft werden lieferantenseitige Änderungen der Materialzusammensetzung nicht kommuniziert, da die Änderung als wenig relevant betrachtet wird. Fehler im Produktionsprozess sind oft komplex und multikausal. Daher lässt sich der Nachweis, dass ein bestimmter mangelhafter Rohstoff ursächlich für einen Fehler ist, im Nachhinein meist nur schwer führen.

Die Übersetzung DE 600 23 005 T2 der europäischen Patentschrift EP 1 194 762 B1 offenbart ein System, eine Einrichtung und ein Verfahren für eine Mehrfacherfassung einer Kombination sowohl chemischer als auch physikalischer Merkmale eines bestimmten Analyten in einer bestimmten Umgebung zur Erzeugung einer eindeutigen Signatur. Ferner kann ein unbekannter Analyt durch Vergleich seiner unbekannten Signatur mit einer gespeicherten Ausgangssignatur bekannter Analyten oder einer Mischung aus Substanzen detektiert und identifiziert werden. Ein Messdatensatz wird durch Algorithmen ausgewertet. Der Algorithmus kann auch die Merkmalsauswahl bzw. Sensorauswahl optimieren. Der Algorithmus kann ein neuronales Netz sein, das durch Korrigieren der falschen oder unerwünschten Ausgänge auf der Grundlage von vorbestimmten Kriterien in einer Sammlung von Daten trainiert wird. U.a. werden in Korrelation stehende Variable in eine kleinere Anzahl an nicht in Korrelation stehenden Variablen transformiert (Hauptkomponentenanalyse, PCA). Ziel der PCA ist die Reduktion der Dimensionalität des Datensatzes. Als Anwendungen sind u.a. die Materialqualitätssteuerung, Arbeiterschutz oder die Überwachung von Nahrungsmittel- und landwirtschaftlichen Produkten genannt. Nicht offenbart ist die Materialanalyse hinsichtlich makroskopischer Materialeigenschaften, wie Teilchengröße, Größenverteilung, Aggregatszustand, rheologischer Eigenschaften oder Mischungsverhältnis von pulverförmigen, heterogenen Materialien. Ferner ist nicht offenbart, wie die automatisierte Materialqualitätssteuerung kontinuierlich betrieben oder in bestehende Produktionsabläufe integrierbar ist. Die Signatur eines Stoffes ist zudem nicht prozess- oder maschinenkorreliert.

Die Übersetzung DE 694 17 519 T2 der europäischen Patentschrift EP 0 606 121 B1 offenbart ein Gerät zur automatischen Qualitätsprüfung ausgewählter Messgrößen pulverförmiger Erzeugnisse. Eine Vielzahl von Prüfeinrichtungen und Datenstationsrechnern erfassen diese Messgrößen parallel an Teilmengen des Pulvers. Messgrößen sind u.a. die Teilchengröße, die Schüttdichte oder der Polymerisationsgrad. Die so gewonnenen Messdaten werden in einer Zentraldatenverarbeitung zusammengeführt und mit Qualitätsstandards verglichen. Auch Fremdstoffe werden bestimmt und mengenmäßig erfasst. Die Auswahl der Messgrößen ist hingegen nicht automatisiert. Es ist ferner kein Algorithmus vorgesehen zur Ermittlung eines reduzierten oder minimalen Satzes an Messgrößen, die zur eindeutigen Probenbestimmung hinreichen. Weiterhin werden die zu vermessenden Teilmengen in Behältern mit Roboterarmen je Behälteraufschrift einer entsprechenden Messanordnung zugeführt. Bei den Messungen und ihren Auswertung bleiben Umgebungsbedingungen unberücksichtigt.

Die deutsche Patentschrift DE 33 08 998 A1 offenbart ein Gerät speziell zur Messung der dispersen Eigenschaften von Pulvern oder Aerosolen. Chemische Messgrößen werden nicht erhoben. Auch werden die Pulver nicht identifiziert. Eine digitale Datenerfassung und -auswertung ist nicht beschrieben.

Die internationale Patentanmeldung WO 99/61902 A1 offenbart eine Methode und ein System zur Identifizierung von Analyten und zur Unterscheidung von anderen Analyten. Analyten im Sinne der Erfindung sind vor allem in Dampf oder Flüssigkeiten getragene Gerüche. In bestimmten Fällen wird eine statistische Metrik verwendet. Eine Vielzahl an d Sensoren werden je verschiedenen Gerüchen ausgesetzt und erzeugen entsprechende elektrische Antworten die als Vektor in einem d-dimensionalen Raum dargestellt werden. Eine statistische Metrik wird in einem zentralen Computer auf Trennschärfe zwischen einem ersten und zweiten Geruch sowie einer möglichst kleinen Anzahl von Detektoren optimiert. Mathematisch umgesetzt wird dies durch die Bestimmung der Projektsachse auf einen möglichst niederdimensionalen Subraum des d-dimensionalen Raumes, welche durch eine optimale Trennung von Datensätze zweier verschiedener Gerüche aufweist. Beschriebene Sensorarten umfassen u.a. akustische, chemische und micro-opto-electro-mechanische Vorrichtungen. Die Antwort aller Sensoren in Kombination schafft einen "Fingerabdruck", der zur Identifikation des Analyten verwendet werden kann. Die Untersuchungsmethoden beziehen sich auf chemische Eigenschaften, nicht hingegen auf physikalische, insbesondere rheologische Eigenschaften. Zwar dient die Reduzierung des Satzes an Sensoren der schnelleren, effizienteren Messung, jedoch wird keine Integration in einen Produktionsprozess oder eine produktionsbegleitende Echzeitmessung offenbart. Der "Fingerabdruck" wird ferner nicht in manipulationssicheren Kennzeichnungen eines Stoffes verwendet. Diese Signatur eines Stoffes ist zudem nicht prozess- oder maschinenkorreliert.

Die U.S. Patentschrift US 5,074,158 A offenbart eine Vorrichtung zur kontinuierlichen Prüfung eines Pulvers, welches durch ein Rohr einer Produktionsanlage geführt wird. Das Prüfergebnis wird durch optische Detektion gewonnen, als Bild auf einem Monitor angezeigt und von einem Bildanalysator ausgewertet.Eine Identifikation des Pulvers ist nicht vorgesehen. Ferner werden die Messdatensätze nicht verknüpft oder durch computergestützte Algorithmen ausgewertet.

Aufgabe der Erfindung ist, ein System aus mehreren Messvorrichtungen zur systematischen, schnellen, kostengünstigen, zuverlässigen und trennscharfen Identifizierung und Diskriminierung verschiedener heterogener Materialien bereitzustellen, das die Trennschärfe der einzelnen Messvorrichtungen steigert und gleichzeitig deren Messaufwand senkt.

Diese Aufgabe wird durch ein System gelöst, das die Merkmale des Anspruchs 13 umfasst.

Weiterhin ist es Aufgabe der Erfindung, ein schnelles, kostengünstiges, zuverlässiges Verfahren zum Betrieb eines Systems zur Identifizierung und Diskriminierung verschiedener heterogener Materialien zu schaffen, das die Trennschärfe steigert und gleichzeitig den Messaufwand senkt.

Diese Aufgabe wird durch ein Verfahren gelöst, das die Merkmale des Anspruchs 1 umfasst.

Die Erfindung betrifft ein System zur Identifizierung oder Diskriminierung von Materialien. Das System besteht zumindest aus mindestens einer lokalen Einrichtung und mindestens einer Zentrale. Jede lokale Einrichtung umfasst mindestens eine Messvorrichtung zur Erfassung je mindestens einer Ist-Signatur für zu bestimmende Materialien und mindestens einen mit der mindestens einen Messvorrichtung kommunikativ verbundenen lokalen Computer mit einer lokalen Datenbasis zur Speicherung und/oder Verarbeitung der Ist-Signatur. Die mindestens eine Zentrale umfasst einen Server mit einer zentralen Datenbank zur Speicherung und/oder Verarbeitung der Ist-Signaturen der lokalen Einrichtung. Das System umfasst ferner ein Netzwerk, das die lokalen Computer der mindestens einen lokalen Einrichtung über den Server der Zentrale kommunikativ verbindet.

Erfindungsgemäß erfolgt die Verarbeitung der Ist-Signaturen nach dem nachfolgend beschriebenen Verfahren bzw. Analyseverfahren. Insbesondere können die Messvorrichtungen der lokalen Einrichtungen des Systems hierzu eine jeweilige Auswahl von u Messapparaturen zur Ausführung von l unterschiedlichen Messmethoden zur Erfassung einer Ist-Signatur aus k zeitabhängigen Messwerten an zumindest einer Probe eines Materials umfassen. Die Auswahl wird aus v Messapparaturen zur Ausführung von m unterschiedlichen Messmethoden zum Bestimmen je einer erweiterten Signatur aus n Messwerten für mindestens ein Soll-Material getroffen. Die Zahlen u, l und k kleiner gleich v, m und n respektive. Eine Datenerfassungsvorrichtung ist mit den u Messapparaturen zur synchronisierten Erfassung der k zeitabhängigen Messwerte der Ist-Signatur und dem jeweiligen lokalen Computer elektrisch verbunden.

Das System kann insgesamt alle Messapparaturen zur Ausführung aller m unterschiedlicher Messmethoden zum Bestimmen je einer erweiterten Signatur aus n Messwerten für mindestens ein Soll-Material umfassen. Folglich können die erweiterten Signaturen durch das System erhoben und nicht-flüchtig in der zentralen Datenbank gespeichert werden. Hierzu ist es nicht erforderlich, dass sämtliche der lokalen Einrichtungen über alle Messapparaturen verfügen. Auch die Zentrale kann mindestens eine weitere Messvorrichtung mit allen Messapparaturen oder nur einer Auswahl daraus umfassen.

Die Erfindung betrifft ferner ein Verfahren zum Betrieb eines Systems zur Identifizierung oder Diskriminierung von Materialien. Das Verfahren umfasst folgende Schritte:
Das Erheben einer Ist-Signatur an einem zu bestimmenden Material durch mindestens einen Benutzer einer lokalen Einrichtung des Systems; die Anfrage des mindestens einen Benutzers an einen Betreiber einer Zentrale des Systems, wobei eine Ist-Signatur ein Vektor aus k unterschiedlichen Messwerten ist, die durch Durchführung l unterschiedlicher Messmethoden (S1 ,...,SI) an dem zu bestimmenden Material erhalten werden, und wobei bei der Anfrage die Ist-Signatur übermittelt wird; das nicht-flüchtige Speichern der vom mindestens einen Benutzer übermittelten Ist-Signaturen auf einem Server der Zentrale; das Erzeugen je einer abgeleiteten Ist-Signatur durch Anwenden je einer durch eine Matrix gegebenen Korrelation für mindestens ein Soll-Material auf die Ist-Signatur. Die Korrelation wird durch derartige Optimierung erhalten, dass eine Abweichung zwischen einer erweiterten Soll-Signatur des jeweiligen Soll-Materials und einer abgeleiteten Soll-Signatur des jeweiligen Soll-Materials minimiert wird. Die erweiterte Signatur besteht wiederum aus n unterschiedlichen Messwerten für das Soll-Material, die mit m unterschiedlichen Messmethoden (S1,...,Sm) erhalten wurden. Die Zahl n ist größer oder gleich der Zahl k und die Zahl m größer gleich der Zahl l, wobei die abgeleitete Soll-Signatur durch Anwenden der Korrelation auf eine Soll-Signatur für das jeweilige Soll-Material erhalten wird, und wobei die Soll-Signatur für das jeweilige Soll-Material ein Vektor aus k Messwerten für das Soll-Material ist, die durch Durchführung der l unterschiedlichen Messmethoden an dem Soll-Material erhalten wurden. Anschließend erfolgt das Berechnen je einer Abweichung der abgeleiteten Ist-Signatur für das Material von der abgeleiteten Soll-Signatur des mindestens einen Soll-Materials. Über das Rückmelden an den anfragenden Benutzer wird mitgeteilt, für welche Soll-Materialien die jeweilige Abweichung eines Materials bezogen auf ein Soll-Materials kleiner als eine für das Soll-Material festgelegte Toleranz ist.

Diese Ist-Signaturen können von mindestens einem Benutzer und/oder vom Betreiber mit auf den anfragenden Benutzer und/oder auf den Betreiber bezogenen Metadaten verknüpft werden. Beispielsweise können der Betreiber oder die Benutzer untereinander im Sinne eines "peerreview" die Messdaten einer Ist-Signatur verifizieren und das Ergebnis als Metadatum mit der Ist-Signatur verknüpfen und übermitteln. Weitere Metadaten umfassen: Eine Benutzungszeit, zu der die Ist-Signatur bzw. deren Messwerte erhoben wurden; eine Messdauer für jede der u Messapparaturen dieser Erhebung; eine Benutzerkennung des die Erhebung ausführenden Benutzers; einen Benutzungsort; eine Kennzeichnung des Materials (z.B. die vermeintliche, zu überprüfende Materialzusammensetzung); eine Kennzeichnung einer Probe oder Charge des Materials; ein Gewicht einer vermessenen Probe des Materials; je eine Kennzeichnung der u Messapparaturen; und/oder Bemerkungen eines Benutzers beispielsweise bezüglich der Messumstände (Umgebungstemperatur, Luftfeuchtigkeit, Dauer und Ereignisse während der Lagerhaltung des Materials) oder der Messziele und Spezifikationen für die Anwendung durch den anfragenden Benutzer. Ein weiteres Metadatum kann eine Klassifizierung der Vertrauenswürdigkeit des anfragenden Benutzers als Datenquelle betreffen. Die Metadaten können insbesondere ein Prüfprotokoll umfassen. Dieses Prüfprotokoll umfasst wenigstens die jeweilige Abweichung und/oder ein Zertifikat über die Qualität des Materials, den Zertifikatgeber und/oder die gemessenen Ist-Signaturen. Der anfragende Benutzer kann dem Betreiber rückmelden, ob die Identifizierung oder Diskriminierung des Materials seines Wissens und seiner Einschätzung nach korrekt ist. Die Rückmeldung erfolgt vorzugsweise in Form von elektronischen Metadaten.

Die Rückmeldung des Betreibers an den anfragenden Benutzer kann zudem auf dessen Bedürfnisse personalisiert werden. Für jeden Benutzer und für mindestens ein Soll-Material kann der Betreiber die zugehörige Korrelation und die zugehörige abgeleitete Soll-Signatur nach einer Kombination von Optimierungskriterien mit zugehörigen Wichtungen optimieren. Vorzugsweise nimmt diese Optimierung ein Algorithmus vor, der auf dem Server der Zentrale des Systems ausgeführt wird. Die Wichtungen werden z.B. anhand von Metadaten modifiziert.

Je öfter das erfindungsgemäße Verfahren ausgeführt wird, und je mehr Benutzer teilnehmen, desto mehr werden die zentralen oder die lokalen Datenbanken erweitert, trainiert und/oder personalisiert. Der Betreiber und/oder ein Benutzer können anhand der Metadaten und/oder der bereits gemessenen Ist-Signaturen diese Ist-Signaturen um durch zusätzliche Messmethoden zu erfassende Messwerte erweitern. Dies bedingt eine neue Optimierung der abgeleiteten Soll-Signaturen und die Korrelationen für mindestens ein Soll-Material. Alternativ kann auch ein bisher weder auf den lokalen noch auf dem zentralen Datenbanken verzeichnetes Material als neues Soll-Material anlegt werden.

Das erfindungsgemäße Analyseverfahren dient der Identifizierung und Diskriminierung von insbesondere heterogenen Materialien. Beispielsweise kann es in einem System zur Identifizierung und Diskriminierung von heterogenen Materialien oder einem System zur Produktbearbeitung eingesetzt werden. Das System zur Produktbearbeitung ist beispielsweise ein Plasmabeschichtungssystem, das unter Verwendung von Pulver, das in einer Plasmastrom eingebracht wird, eine Beschichtung auf eine Materialoberfläche aufbringt. Es kann beispielsweise auch zur Qualitätskontrolle oder bei der Materialsteuerung eingesetzt werden. Bei dem Analyseverfahren werden zunächst unterschiedliche m Messmethoden auf ein oder mehrere Soll-Materialien angewendet. Ein Soll-Material zeichnet sich dadurch aus, dass es für die Produktbearbeitung geeignet ist. Jede der m Messmethoden erfasst zumindest eine Messgröße. Alle für ein Soll-Material erfassten Messgrößen werden erfindungsgemäß in einem Datenvektor aggregiert, der mindestens aus n=m Messwerten besteht. Die Dimension des zugehörigen Vektorraums entspricht der Anzahl n der Messwerte. Diese Dimension korreliert stark positiv mit der Trennschärfe des Analyseverfahrens. So können bereits geringfügige Änderungen in einem Material sicher detektiert werden. Aus jedem dieser Datenvektoren wird je eine erweiterte Signatur ("Fingerabdruck") erzeugt. Die m Messmethoden sind so ausgewählt, dass die erweiterte Signatur charakteristisch für das Soll-Material ist, also im mathematischen Sinne eindeutig bestimmt oder überstimmt ist. In einem weiteren Schritt wird aus den Soll-Signaturen aller Soll-Materialien eine Datenbasis erstellt.

In einem darauffolgenden Optimierungsschritt wird für jedes Soll-Material eine Auswahl von l Messmethoden aus den m Messmethoden getroffen, wobei die Zahl l kleiner oder gleich der Zahl m ist. Entsprechend erzeugt diese Auswahl von l Messmethoden Soll-Signaturen. Als Datenvektoren notiert haben diese Soll-Signaturen eine niedrigere oder maximal gleiche Dimension als die vom vollen Satz von m Messmethoden erzeugten erweiterten Soll-Signaturen. Diese Soll-Signaturen werden entweder erneut gemessen oder durch Weglassen derjenigen n-k Messwerte erzielt, welche die l Messmethoden nicht erfasst haben. Für jedes Paar aus erweiterter Soll-Signatur und Soll-Signatur wird ferner eine Korrelation ermittelt. Durch Anwendung dieser Korrelation auf die Signatur wird eine abgeleitete Soll-Signatur erzeugt. Die Auswahl der l Messmethoden und die zugehörige Korrelation werden iterativ optimiert. Beispielsweise wird die Anzahl oder der Gesamtmessaufwand der Messmethoden minimiert.

Im Folgeschritt wird eine Soll-Datenbasis erstellt. In ihr sind für jedes Soll-Material ein Satz von abgeleiteten Soll-Signaturen, Auswahlen von Messwerten und zu ihrer Erhebung erforderlicher Messmethoden und/oder Korrelationen enthalten.

In einem weiteren Schritt wird mindestens ein unbestimmtes Materials bezüglich seiner Eignung zum Verarbeiten in einer Vorrichtung zur Produktbearbeitung überprüft. Dazu wird jeweils einelst-Signaturen gemessen; dann wird je eine abgeleitete Ist-Signatur erzeugt, indem für jedes relevanten Soll-Materials diejeweilige Korrelation auf die Ist-Signatur angewandt wird; anschließend wird jeweils die Abweichung der abgeleiteten Ist-Signaturen jedes Materials von der abgeleiteten Soll-Signatur des mindestens einen Soll-Materials ermittelt; letztlich wird Abweichung mit einer Toleranz verglichen, die für die Vorrichtung zur Produktbearbeitung, das Soll-Material und/oder das herzustellende Produkt spezifiziert ist. Die Abweichungen werden vorzugsweise aus der Vektornorm der Differenz zwischen abgeleiteter Soll-Signatur eines Soll-Materials und der abgeleiteten Ist-Signatur eines Materials ermittelt.

Ist die Abweichung für ein Material kleiner als die Toleranz, wird es in einem letzten Schritt in der Vorrichtung zur Produktbearbeitung verarbeitet, für die Verarbeitung freigegeben oder die Verarbeitung eines freigegebenen Materials protokolliert.

Zu den zu untersuchenden Materialeigenschaften und Messgrößen zählen insbesondere, die Zusammensetzung des Materials oder seiner Oberfläche die rheologischen Eigenschaften, die elektromagnetischen Eigenschaften, die mechanischen Eigenschaften. die optischen Eigenschaften,die Schüttdichte, die Körnigkeit des Materials, die Größenverteilung der Teilchen des Materials, die geometrischen Eigenschaften der Teilchen, die magnetische Suszeptibilität, die Hochfrequenzeigenschaften, die elektrische Leitfähigkeit, die Sublimations- oder Schmelztemperatur, das Gewicht und/oder spezifisches Gewicht des Materials, oder die komplexe Impedanz unter Wechselspannungsbeaufschlagung. Des Weiteren können mehrere Messgrößen in ihrer Anhängigkeit zueinander erfasst werden, beispielsweise die auf das Material einwirkende Presskraft als Funktion des Presswegs. Aus dem zeitlichen Verlauf solcher Abhängigkeiten der verschiedenen Messwerte können wenige charakteristische abgeleitete Messwerte bzw. Parameter bestimmt werden.

Zu den erfindungsgemäß verwendeten und kombinierten Messmethoden zählen insbesondere chemische Messmethoden; rheologische Messmethoden; optische Messmethoden; hochauflösende Farbbilderphotographie; thermodynamische Messungen; Röntgenbeugung, Bestimmung der magnetischen Stoffeigenschaften; Erfassung geometrischer Verteilungsfunktionen und Datenanalyseverfahren zur Extraktion abgeleiteter Messwerte bzw. Parameter. Eine gegebene Messmethode kann durch verschiedene, aufeinander kalibrierte Messapparaturen durchgeführt werden. Eine Kombination von mehreren Messmethoden ist besonders bei heterogenen Materialmischungen jeder Einzelmethode bezüglich der Trennschärfe weit überlegen.

Der Optimierung eines Satzes von abgeleiteten Soll-Signaturen, Auswahlen und/oder Korrelationen für jedes Soll-Material kann insbesondere mittels eines computergestützten Algorithmus vorgenommen werden. Der Algorithmus kann beispielsweise ein künstliches neuronales Netz (KNN), ein genetischer Algorithmus, eine Hauptkomponentenanalyse, ein Monte-Carlo-Algorithmus, ein Simulated Annealing-Algorithmus, ein Fuzzy-Logik-Algorithmus oder ein K-Nearest-Neighbour-Algorithmus sein.

Der Optimierung können erfindungsgemäß nach einer Kombination von Optimierungskriterien erfolgen. Die Optimierungskriterien können zudem gemäß ihrer Relevanz für das Verarbeiten eines Materials in einer Vorrichtung zur Produktbearbeitung mit zugehörigen Wichtungen versehen werden.

Zu den Optimierungskriterien können zählen: Eine für das Verarbeiten in der Vorrichtung zur Produktbearbeitung hinreichend kleine erste Abweichung zwischen der Signatur und der abgeleiteten Signatur für ein jeweiliges Soll-Material; die Relevanz der Eigenschaften eines Soll-Materials für das Verarbeiten in der Vorrichtung zur Produktbearbeitung; die Relevanz der durch die Messmethoden erhobenen Messwerte für das Verarbeiten in der Vorrichtung zur Produktbearbeitung; die Messzeit jeder der l Messmethoden sowie ihre kumulierte Messzeit unter Berücksichtigung, dass manche Messmethoden parallel ausgeführt werden können; die Messkosten der l Messmethoden und des mit einer Messmethode verbundenen apparativen Aufwands; die Integrierbarkeit der Messmethoden in den Prozess zur Produktbearbeitung; die Spezifikationen des bearbeiteten Produkts; die Umgebungseinflüsse auf die Vorrichtung zur Produktbearbeitung; die statistische Plausibilität der Messwerte zur Vermeidung von "Ausreißern". Ein Messwert kann im Sinne der Erfindung das Ergebnis einer einzelnen Messung oder ein statistisch aussagekräftiges Daten-Ensemble aus wiederholten Messungen sein.

Es ist meist zu aufwendig, in laufenden Fertigungsprozessen zur Qualitätssicherung mehrere komplexe Methoden ständig oder auch stichprobenartig parallel zum Fertigungsprozess durchzuführen.

Durch die Kombination von Messmethoden, die kostengünstig und schnell sind und am laufenden Prozess kontinuierlich oder stichprobenartig mit hoher Frequenz durchgeführt werden, mit Methoden die aufwändig, zeit- und kostenintensiv sind und für jede Materialzusammensetzung nur selten oder einmalig durchgeführt werden, ist eine Materialcharakterisierung mit hoher Trennschärfe gegeben.

Am Beispiel eines Simulated Annealing-Algorithmus kann die Optimierung nach mehreren Optimierungskriterien so erfolgen: Es wird eine Gesamtenergiefunktion bestimmt. Jedes Optimierungskriterium liefert einen Energiebeitrag. Je nach Relevanz des Optimierungskriteriums wird der Energiebeitrag gewichtet. Beispielweise könnte der Schmelzpunkt eines Materials irrelevant für einen Produktionsprozess sein. Sein Energiebeitrag wird entsprechend sehr gering oder mit dem Faktor Null gewichtet. Falls die Messzeit oder -kosten einer jeweiligen Messmethode prozesskritisch sind, wird der entsprechende Energiebeitrag entsprechend hoch gewichtet. Die Gesamtenergiefunktion kann gegen die Optimierungskriterien als Variable wie eine hochdimensionale Energielandschaft aufgetragen werden. In dieser Energielandschaft ermittelt der Algorithmus ein lokales Minimum, das einem optimierten Parametersatz entspricht. Der Optimierungsvorgang erfolgt über eine Iteration von Zufallsschritten. Mit steigender Energiedifferenz zwischen Ausgangs- und Zielparametersatz des Zufallsschritts wird der Zielparametersatz mit geringerer Wahrscheinlichkeit angenommen. Folglich wird nicht unbedingt der global beste, sondern nur ein für den Produktionsprozess hinreichend geeignete Parametersatz ermittelt. Wenn viele Materialeigenschaften nicht oder wenig prozessrelevant sind, wird zudem eine breite Klasse prozessgeeigneter Materialien um das der Optimierung zugrunde liegende Soll-Material definiert. Werden hingegen alle Materialeigenschaften hoch gewichtet, werden im Grenzfall alle Messmethoden ausgewählt (l=m), so dass die Trennschärfe der Materialidentifikation und -diskriminierung zu Lasten der Messzeit oder der Messkosten steigt.

In einer Ausführungsform der Erfindung kann eine ausgewählte Untermenge von Soll-Datensätzen in einer Datenbasis gespeichert werden. Die Datenbasis enthält Soll-Datensätze, welche jeweils aus der Auswahl der Messmethoden, der Soll-Signatur, der Korrelation und/oder der abgeleiteten Soll-Signatur des mindestens einen Soll-Materials bestehen. Desgleichen können in der Datenbasis während des Verarbeitens eines Materials Prüfdatensätze protokolliert werden, welche aus den Ist-Signaturen, den abgeleiteten Ist-Signaturen und/oder den Abweichungen bestehen.

In einer weiteren erfindungsgemäßen Ausführungsform werden die Prüfdatensätze in der Datenbasis automatisch über ein digitales Netzwerk an eine weitere, externe Datenbasis übermittelt werden. Diese externe Datenbasis kann beispielsweise im Herrschaftsbereich der Produktionskontrolleure oder des Herstellers der Vorrichtung zur Produktbearbeitung liegen. Aus Schutz vor Manipulation können die Prüfdatensätze verschlüsselt übermittelt werden. Der Speicherort der externen Datenbasis kann örtlich beliebig liegen und beispielsweise via Funk oder das Internet mit der Vorrichtung zur Produktbearbeitung verbunden sein.

Des Weiteren können die Prüfdatensätze auf der Datenbasis auch als Steuerparameter für einen Regelkreis beim Verarbeiten in einer Vorrichtung zur Produktbearbeitung verwendet werden.

Ferner können auf einem externen Speichermedium (vom System unabhängig) ausgewählte Solldatensätze und/oder Prüfdatensätze gespeichert sein. Dieses externe Speichermedium wird mit einem Gebinde, einer Verpackungen und/oder einer Charge eines Soll-Materials oder eines Materials verbunden. Die Daten können mit entsprechenden Mitteln ausgelesen werden. Ein Soll-Datensatz auf dem externen Speichermedium gibt beispielsweise an, mit welchem Soll-Material anhand seines zugehörigen Solldatensatzes ein Material abgeglichen werden soll. Ein Prüfdatensatz auf dem externen Speichermedium fungiert beispielsweise als "Fingerabdruck" für ein Material.

Das externe Speichermedium kann aus einem Radio-Frequency Identification (RFID)-Chip bestehen, auf dem Datensätze in Form von RFID-Tags gespeichert werden. Zusätzlich oder alternativ kann das externe Speichermedium auch aus einem Etikett bestehen, auf dem Datensätze alphanumerisch und/oder als Barcode gespeichert werden.

In einer besonderen Ausführungsform ist die Auswahl der l Messmethoden darauf optimiert, dass die Eignung eines oder mehrerer Materialien für das Verarbeiten in der Vorrichtung zur Produktbearbeitung während des laufenden Produktionsprozesses überprüft werden kann. Ideal wäre eine kontinuierliche Überprüfung, wobei die Überprüfung, gemessen an den typischen Prozesszeiten, kurzen Intervallen oder in Echtzeit erfolgt. Zur Beschleunigung des gesamten Messverfahrens können verschiedene Messmethoden verschiedene Stichproben eines Materials parallel untersuchen.

Gemäß der Erfindung ist das Anwenden der Auswahl der k Messmethoden mindestens zehnmal schneller als das Anwenden der m Messmethoden.

Ferner kann eine Datenverarbeitungseinrichtung die Prüfdatenbasis auslesen und sicherstellen, dass in der Prüfdatenbasis enthaltene Messwerte nicht redundant erhoben werden. Zu einer redundanten Erhebung kann es kommen, wenn ein Messwert an einem Material zum Vergleich mit mehreren Soll-Materialien zu messen ist. Ist ein Messwert bereits in der Prüfdatenbasis enthalten, so steuert die Datenverarbeitungseinrichtung den reduzierten Satz von u Messapparaturen zum Durchführen der Auswahl der Messmethoden auf ein Material derart an, dass der Messwert nicht nochmals erhoben wird.

Die Erfindung umfasst weiterhin ein System zur Produktbearbeitung. Es enthält eine Vorrichtung zur Produktbearbeitung zur Verarbeitung mindestens eines unbestimmten, heterogenen Materials. Das System ist weiterhin gekennzeichnet durch einen Satz von u Messapparaturen zur Ermittlung je einer Ist-Signatur für das mindestens eine Material. Ferner umfasst es ein Datenverarbeitungssystem, das mit dem Satz von u Messapparaturen kommunikativ verbunden ist. Das Datenverarbeitungssystem ist zur Ausführung folgender Operationen ausgelegt: Der Speicherung je einer abgeleiteten Soll-Signatur und je einer Korrelation für mindestens ein Soll-Material, welches für die Produktbearbeitung geeignet ist; der Anwendung der Korrelation für jedes Soll-Material auf die Ist-Signatur jedes Materials zur Berechnung je einer abgeleiteten Ist-Signatur; sowie der Speicherung eines Protokolls aus Prüfdatensätzen. Die Prüfdatensätze bestehen zumindest aus der Abweichung zwischen den abgeleiteten Soll-Signaturen und der jeweiligen abgeleiteten Ist-Signatur. Zusätzlich oder alternativ kann das System auch eine Steuereinheit umfassen, die mit dem Datenverarbeitungssystem kommunikativ verbunden ist. Die Steuereinheit kann Steuersignale aus den Prüfdatensätzen generieren und diese an die Vorrichtung zur Produktbearbeitung übermitteln.

Zusätzlich zum beschriebenen System kann ein externes System vorgesehen sein. Das externe System umfasst einen Satz von v Messapparaturen, mit welchem je eine charakteristische Signatur für das mindestens eine Soll-Material erzeugt wird. Die Zahl vist hierbei größer oder gleich der Zahl u. Ferner umfasst das externe System einen Computer, mit welchem aus den Signaturen mittels eines Algorithmus die Soll-Datensätze erzeugbar sind. Die Soll-Datensätze bestehen ihrerseits aus abgeleiteten Ist-Signaturen, einer Korrelation zu einer Ist-Signatur und/oder der Ist-Signatur selbst. Ferner umfasst das externe System ein Speichermedium zur Speicherung der Soll-Datensätze und eine Datenverbindung zur Übermittlung ausgewählter Soll-Datensätze an das Datenverarbeitungssystem oder das erste Speichermedium. Das externe System kann zeitlich und örtlich unabhängig vom System zur Materialbearbeitung betreibbar ausgeformt sein.

In einer besonderen Ausführungsform kann das Datenverarbeitungssystem ein externes Speichermedium umfassen. Auf dem externen Speichermedium sind ausgewählte Soll-Datensätze für mindestens ein Soll-Material und das Protokoll aus Prüfdatensätzen ausgewählter Materialien abgelegt. Ferner umfasst das Datenverarbeitungssystem eine Datenverarbeitungseinrichtung zur Anwendung einer Korrelation eines Sollmaterials auf eine Ist-Signatur eines gegebenen Materials.

Das externe Speichermedium kann mit einem Gebinde, einer Verpackungen und/oder einer Charge eines Soll-Materials oder eines Materials auslesbar verbunden sein. Es kann somit Informationen enthalten, wie ein gegebenes Material zu prüfen ist. Ebenso kann auf ihm ein Prüfergebnis als "Fingerprint" gespeichert sein.

Insbesondere kann das externe Speichermedium als ein auslesbarer und/oder beschreibbarer Radio-Frequency Identification (RFID)-Chip ausgeführt sein. In diesem Fall umfasst das Datenverarbeitungssystem ein Gerät mit einer geeigneten, frequenzabstimmbaren Antenne zum Auslesen und Editieren sowie Beschreiben mit Daten auf dem RFID-Chip.

Über eine Datenfernverbindung können zusätzlich zu Zwecken der manipulationssicheren Fernüberwachung der Produktion die Prüfdatensätze vom Datenverarbeitungssystem an ein weiteres externes Speichermedium übermittelt werden. Die Daten können insbesondere verschlüsselt gesendet werden. Als Datenfernverbindung können beliebige Funkstandards (z.B. per SMS) oder das Internet genutzt werden.

Die Erfindung eignet sich besonders für Beschichtungsverfahrenvon Substraten. Besonders geeignete Materialien zu Beschichtung sind beispielsweise pulverförmige Legierungen, Pulver, Pulvermischungen, ein Granulate, Pasten, Lösungen, Suspensionen oder Dispersionen oder eine Mischung daraus.

In einer weiteren Ausführungsform der Erfindung ist eine Zufuhreinrichtung für das mindestens eine Material in die Vorrichtung zur Produktbearbeitung vorgesehen. Zunächst wird das Ergebnis des Vergleichs aus der Abweichung und einer für die Vorrichtung zur Produktbearbeitung und einer das Soll-Material spezifizierten Toleranz ermittelt und an die Steuereinheit kommuniziert. Die Zufuhreinrichtung steht mit der Steuereinheit in kommunikativer Verbindung. In Abhängigkeit dieses Ergebnisses regelt die Steuereinheit den Materialstrom durch die Zufuhreinrichtung.

Eine Messvorrichtung zur Erfassung von Materialeigenschaften von Materialien, die selbst nicht zur Erfindung gehört, aber insbesondere in den lokalen Einrichtungen und der Zentrale des vorbeschriebenen Systems zur Identifizierung und Diskriminierung von Materialien und zur Ausführung der erfindungsgemäßen Verfahren eingesetzt wird, wird nun beschrieben. Die Messvorrichtung umfasst mindestens einen Prüfkörper zur Aufnahme je einer Probe des Materials. Dem Prüfkörper sind mehrere Messapparaturen zugeordnet. Die Messapparaturen dienen der Ausführung von l unterschiedlichen Messmethoden an der Probe, um eine Ist-Signatur des Materials aus mindesten l zeitabhängigen Messwerten zu erfassen. Die Messapparaturen umfassen wenigstens: Einen Druckstempel und einen Druckmesser zur Erfassung des vom Druckstempel auf die Probe beaufschlagten Drucks P; einen Wegmesser zur Erfassung eines durch den Druckstempel zurückgelegten Weges; und ein elektrisches Messgerät zur Erfassung eines elektrischen Widerstandes R der Probe. Ferner umfasst die Messvorrichtung eine Datenerfassungsvorrichtung, die mit den Messapparaturen zur synchronisierten Erfassung der k zeitabhängigen Messwerte der Ist-Signatur elektrisch verbunden ist.

Insbesondere werden alle Messungen auf eine gemeinsame räumliche Achse bezogen. Dazu werden die Messapparaturen z.B. jeweils so ausgebildet und angeordnet, dass durch den Druckmesser der vom Druckstempel auf die Probe beaufschlagte Druck P, durch den Wegmesser der vom Druckstempel zurückgelegte Weg W und durch das elektrische Messgerät der elektrischen Widerstand R der Probe entlang einer Achse erfasst werden können.

Datenerfassungsvorrichtung kann eine standardisierte Datenschnittstelle, wie einen USB-Port, zur Übertragung der Ist-Signatur an eine Datenverarbeitungseinrichtung aufweisen.

Zusätzlich kann der Satz von u Messapparaturen der Messvorrichtung auch ein optisches Messgerät zur optischen Erfassung einer Oberfläche der Probe, ein aktives oder passives akustisches Messgerät zur Erfassung des akustischen Eigenspektrums bzw. Resonanzen der Probe, ein Thermometer zur Erfassung einer Proben- oder Umgebungstemperatur, eine Waage zur Erfassung eines Gewichts der Probe; und/oder ein Hygrometer zur Erfassung einer Luftfeuchtigkeit in der Messvorrichtung umfassen.

Die die Datenerfassungsvorrichtung kann mit einem lokalen Computerin kommunikativer Verbindung stehen. Der lokale Computer kann die direkten Messwerte, die mit den u Messapparaturen aufgenommen worden sind, aufnehmen, auswerten und speichern. Zum Beispiel kann der lokale Computer eine Messkurve des Drucks in Abhängigkeit des Weges mit einer Sigmoidfunktion fitten und die Fitparameter extrahieren. Diese Fitparameter können statt der direkten Messwerte in die ermittelten Ist-Signaturen eingehen. Ferner kann der lokale Computer der Erfassung auf einen Benutzer der Messvorrichtung bezogene Metadaten dienen.

Das erfindungsgemäße Analyseverfahren und System können zum Untersuchen, Identifizieren oder Diskriminieren von pulverförmigen Legierungen, Pulvern, Pulvermischungen, Granulaten, Pasten, Lösungen, Suspensionen, Dispersionen oder einer Mischung daraus verwendet werden. Ebenso können sie auf mehrere verschiedene Materialien angewendet werden, um Materialmischungen, Legierungen oder Verbundmaterialien durch den Prozess zur Materialverarbeitung protokollierbar und kontrolliert zu erzeugen.

Beispielhaft wird nachfolgend eine Ausführungsform der Erfindung am Beispiel der Auswahl vom l=3 aus m=5 Messmethoden erläutert.

Darin wird eine Sequenz von fünf verschiedenen Messmethoden auf ein Pulver angewendet. Die Korrelation zwischen den Ergebnissen der Messmethoden ist empirisch nicht oder nicht genau bekannt.

Die erste Messmethode ist eine optische Charakterisierung: Das Pulver wird flächig auf eine transparente Platte aufgebracht und dann wird mit einem Flachbettscanner ein optisches Bild aufgenommen. Der Flachbettscanner kann mit einem synchron aufgenommenen Farbnormal kalibriert werden. Aus diesem Bild können charakteristische Daten, wie z.B. die mittleren Farbwerte (RGB o.ä.), sowie abgeleitete Parameter, die räumliche Verteilung dieser Farbwerte beschreiben, gewonnen werden. Ebenso kann die optische Charakterisierung mit einem geeigneten Prüfkörper erfolgen, der hierzu ein transparentes Fenster aufweist und eine ihm zugeordnete Kamera die Farbwerte aufzeichnet.

Die zweite Methode ist eine mechanische Charakterisierung: Eine Probe des Pulvers wird in dem Prüfkörper uniaxial belastet und die Kurve aus Presskraft und Pressweg aufgezeichnet. Eine solche Kurve hat typischerweise einen sigmoiden Verlauf und kann durch wenige Parameter beschrieben werden.

Die dritte Methode ist eine elektrische Charakterisierung: Parallel zur Anwendung der zweiten Methode kann die Abhängigkeit der elektrischen Leitfähigkeit des Pulvers in Abhängigkeit von der Presskraft aufgezeichnet werden. Auch diese Kurve hat einen charakteristischen sigmoiden Verlauf und kann durch wenige Parameter beschrieben werden.

Die vierte Methode ist eine geometrische Charakterisierung: Mit einem statischen mikroskopischen Analyseverfahren wird die Korngrößenverteilung aufgezeichnet. Bei unregelmäßig geformten Partikeln empfiehlt es sich der Einfachheit halber meist, einen Äquivalentdurchmesser zur Charakterisierung der Größe anzugeben. Muss zusätzlich auch noch die Form des Partikels berücksichtigt werden, kann aus zwei Äquivalentdurchmessern einen Formfaktor als Quotient der beiden Werte definiert werden, beispielsweise aus volumenäquivalentem und oberflächenäquivalentem Kugeldurchmesser. Die geometrischen Daten sind z.B. durch einen mittlerem Formfaktor und den Verteilungsparametern D10, D50 und D90 gut beschreibbar.

Die fünfte Methode liefert Information über die chemische Zusammensetzung des Materials im Volumen: Mit einer chemischen Analytik wird z.B. die Elementzusammensetzung des Materials geprüft.

Die erste, zweite und dritte Methode sind schnell und einfach durchführbar. Dagegen erfordern die vierte und fünfte Methode einen erheblichen präparativen und instrumentellen Aufwand. Ein Pulver kann beispielsweise durch eine Signatur, die mit diesen fünf Methoden erzeugt wird, als Eigenschaftsvektor in einem 50-dimensionalen Messwerteraum eindeutig beschrieben werden. Der Unterraum aus den Messwerten der ersten bis dritten Methoden hat dagegen in diesem Beispiel nur 14 Dimensionen.

Obwohl die Messmethoden voneinander verschieden sind, sind ihre Messergebnisse auf komplexe, unbekannte, empirisch schwer zu fassende Weise korreliert. Beispielsweise hängt für ein metallisches Pulver die Färbung, die mit der ersten Methode gemessen wird, mit dem Oxidgehalt bzw. der Oxidation der Oberfläche der Pulverteilchen, der mit der fünften Methode bestimmt wird, zusammen. Analog hat der Oxidgehalt einen Einfluss auf die elektrischen Eigenschaften, die vermittels der dritten Methode gemessen werden.

Aufgrund dieser "versteckten", komplexen Querabhängigkeiten ist es möglich, einen unvollständig gemessenen Eigenschaftsvektor zu ergänzen. Algorithmen z.B. "Künstliche Neuronale Netze (KNN)" eignen sich besonders gut für die Ergänzung des Eigenschaftsraumes. Hierzu wird für ein Material der vollständige Methodensatz einige Male angewendet und daraus der statistisch abgesicherte vollständige Eigenschaftsvektor gewonnen. Das neuronale Netz wird nun so trainiert, dass auch bei Input eines unvollständigen Eigenschaftsvektors als Output ein vollständiger Eigenschaftsvektor rekonstruiert wird. In der Praxis ist es vorteilhaft zur Gewinnung des unvollständigen Eigenschaftsvektors die schnellen und einfachen Messmethoden zu nutzen.

Für die Qualitätssicherung ist es möglich, eine abgeleitete Soll-Signatur und über eine geeignete Metrik eine maximale Toleranz zur gemessenen abgeleiteten Ist-Signatur festzulegen, die für einen gegebenes Produktionsverfahren akzeptabel ist. Je nach Wahl der Toleranz sowie geeigneter Wahl der Messmethoden und der Optimierung der Koeffizienten liefert der Algorithmus bereits auf Basis eines unvollständigen Messdatensatzes, Ist-Signaturen, einen rekonstruierten Eigenschaftsvektor, die abgeleitete Ist-Signatur, welche sich mit hinreichender Trennschärfe von der abgeleiteten Soll-Signatur eines Soll-Materials unterscheidet.

Neben der Qualitätssicherung kann die Methode auch genutzt werden, um einen "Fingerabdruck" für ein Material zu bestimmen. Dieser "Fingerabdruck" kann auf dem Materialgebinde in alphanumerischer Form, oder als RFID-Tag abgespeichert werden und dazu dienen, die Originalität des Materials abzusichern. Die Originalitätsprüfung kann dabei bereits mit dem reduzierten Satz an Messmethoden, wie z.B. der ersten bis dritten Messmethode, und damit einfach und schnell erfolgen.

Insbesondere lassen sich auf diese Weise z.B. Pulvermaterialien manipulationssicher klassifizieren. Die Klassifizierung lässt sich in einem eindeutigen numerischen Wert festhalten. Dieser Wert kann z.B. via RFID-Tag oder Barcode oder auch alphanumerisch auf dem Materialgebinde festgehalten werden, um die Chargenqualität zu sichern oder um zu verhindern, dass bewusst minderwertige Materialien aus unsicherer Quelle eingesetzt werden. Wird der vollständige Eigenschaftsvektor als "Fingerabdruck" auf dem Materialgebinde abgespeichert, kann der Algorithmus sehr allgemein formuliert werden und wird auch bei sehr verschiedenartigen zu diskriminierenden Materialien sehr schnell konvergieren.

Nachfolgend wird ein Ausführungsbeispiel der Erfindung anhand der beigefügten Figuren näher beschrieben. Es zeigen:
- **Figur 1**: eine schematische Darstellung des erfindungsgemäßen Optimierungsverfahrens;
- **Figur 2**: eine schematische Darstellung der gewichteten Optimierungskriterien und der Optimierungsmethode;
- **Figur 3**: eine schematische Darstellung der erfindungsgemäßen Analyseverfahrensschritte zur Identifizierung bzw. Diskriminierung eines Materials aus bzw. gegen Soll-Materialien;
- **Figur 4**: eine schematische Darstellung des erfindungsgemäßen Analyseverfahrens;
- **Figur 5**: eine schematische Darstellung des erfindungsgemäßen Systems zur Produktbearbeitung;
- **Figur 6**: eine schematische Darstellung einer Messvorrichtung zur Erfassung von Materialeigenschaften von Materialien, die nicht Teil der Erfindung ist;
- **Figur 7A**: einen mit einer Probe eines Materialsbefüllten Prüfkörper vor Druckbeaufschlagung durch den Druckstempel;
- **Figur 7B**: einen mit einer Probe eines Materialsbefüllten Prüfkörper nach Druckbeaufschlagung durch den Druckstempel;
- **Figur 7C**: einen mit einer Eichvorrichtung befüllten Prüfkörper;
- **Figur 8**: eine schematische Darstellung eines erfindungsgemäßen Systems zur Identifizierung oder Diskriminierung von Materialien; und
- **Figur 9**: eine schematische Darstellung des Verfahrens zum Betrieb eines erfindungsgemäßen Systems zur Identifizierung oder Diskriminierung von Materialien.

In den Zeichnungen werden für gleiche oder gleich wirkende Elemente der Erfindung identische Bezugszeichen verwendet.

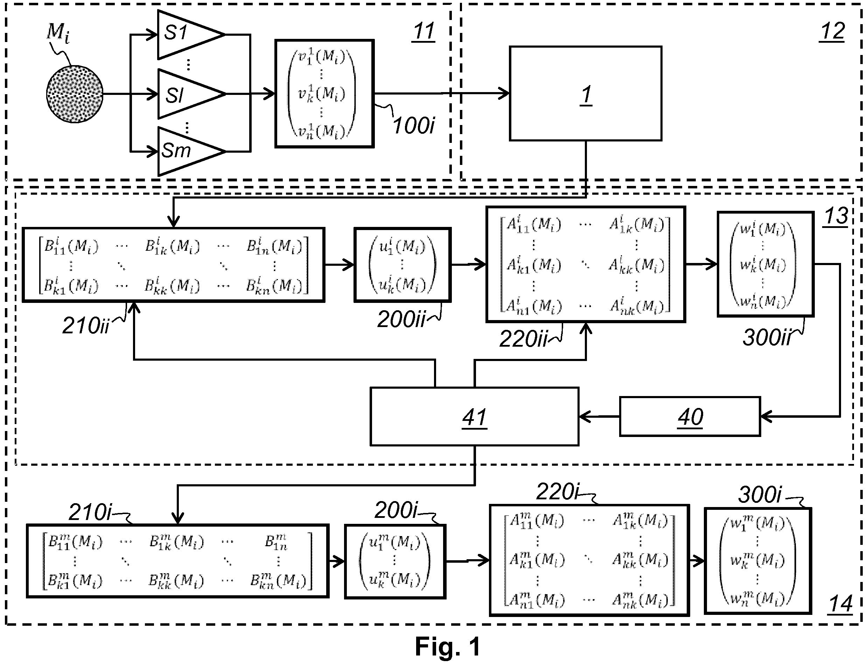

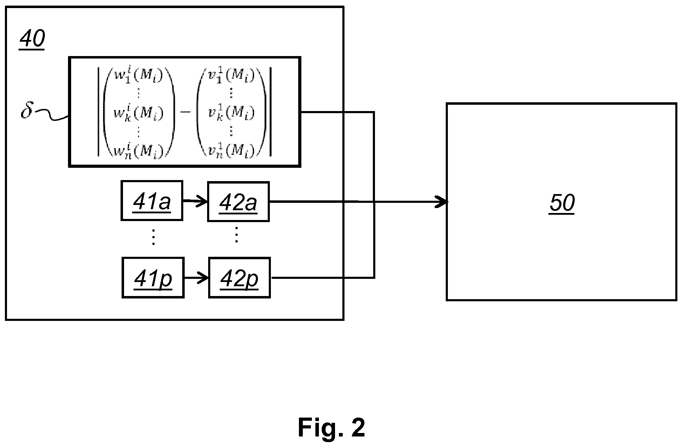

**Figur 1** zeigt eine Ausführungsform des erfindungsgemäßen Optimierungsverfahrens in schematischer Darstellung. Im Schritt 11 werden ein oder mehrere Soll-Materialien Mᵢ ausgewählt und mit einem Satz aus m verschiedenen Messmethoden S1, ..., Sm vermessen. Jede Messmethode S1, ..., Sm bestimmt einen oder mehrere unterschiedliche Messwerte. Jeder Messwert kann mehrmals bestimmt werden und statistisch zu einem abgesicherten Messwert zusammengefasst werden. Zur Datenanalyse werden die Messwerte zu einem Eigenschaftsvektor zusammengefasst. Die m Messmethoden S1, ..., Sm sind so ausgewählt, dass der Eigenschaftsvektor eines Soll-Materials Mᵢ eine eindeutige, charakteristische erweiterte Signatur 100i darstellt. Im Schritt 12 wird aus der erweiterten Signatur 100i für jedes Soll-Material Mᵢ eine Datenbasis 1 erstellt. Im Schritt 13 wird eine zufällige oder auf Empirie beruhende Startauswahl von l der m Messmethoden S1, ...Sm getroffen, wobei l kleiner gleich m ist. Diese wird iterativ optimiert. In der i-ten Optimierungsiteration 13 wird zunächst die i-te Auswahl 210ii von Messmethoden angegeben, vorzugsweise in Matrixnotation. Das Anwenden der Auswahl (-Matrix) 210ii auf die erweiterte Signatur 100i erzeugt eine i-te Soll-Signatur für ein Soll-Material Mᵢ mit geringerer oder gleicher Dimension. Ein Algorithmus erzeugt daraufhin eine i-te Korrelation 220ii, deren Anwendung auf die Signatur eine i-te abgeleitete Signatur 300ii von gleicher Dimensionalität wie die erweiterte Signatur 100i. Anhand einer Kombination 40 (siehe Figur 2) aus Optimierungskriterien 41a, ..., 41p wird durch eine Optimierungsmethode 50 wie z.B. der eines KNN-Algorithmus die i-te Auswahl 210ii und die Korrelation 220ii iterativ optimiert, bis das globale oder ein lokales Optimum ermittelt ist. Die Optimierung 14 z.B. kann durch Erreichen eines festgelegten Konvergenzkriteriums oder nach einer festgesetzten Anzahl von m Optimierungsiterationen 13 beendet werden. Ein Optimum besteht aus einer Auswahl 210i von l Messmethoden S1, ...Sl, einer Soll-Signatur 200i, einer Korrelation 220i und einer abgeleiteten Soll-Signatur 300i für jeweiliges Soll-Material Mᵢ. Diese Auswahl 210i kann eine beliebige Kombination aus den m Messmethoden S1, ..., Sm sein. Desgleichen kann die Anzahl l der ausgewählten Messmethoden S1, ..., Sl bei Startauswahl und optimierter Auswahl 210i verschieden sein. In der Praxis vorteilhaft ist es, in die Auswahl 210i keine Wichtungen und Kreuzverbindungen aufzunehmen. Diese können gleichwertig ausschließlich in die Korrelation 220i eingehen. Das bedeutet, dass die Auswahl 210i als Diagonalmatrix mit Diagonalelementen 0 oder 1 repräsentiert werden kann. In diesem Fall gibt die Auswahl 210i direkt und ohne Rechenaufwand an, welche Messgröße welcher Messmethode S1, ..., Sm zu erheben ist.

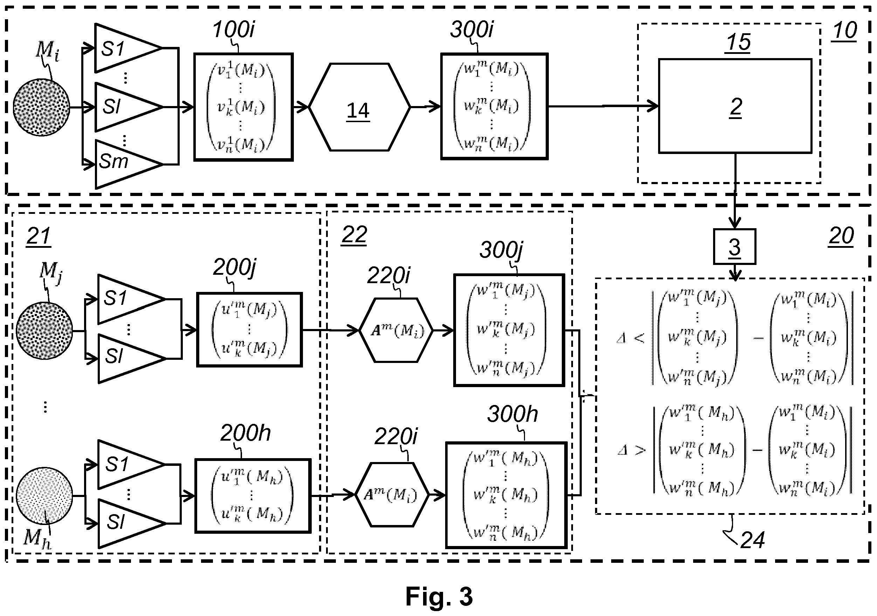

**Figur 2** illustriert die Kombination 40 aus Optimierungskriterien 41a, ..., 41p und wie sie Eingang in die Optimierungsmethode 50 erhalten. Die Auswahl der Optimierungskriterien 41a, ..., 41p hängt erfindungsgemäß von einer Zielsetzung ab. Die Zielsetzung kann z.B. sein, ein Substrat in einer plasmagestützten Beschichtungsanlage mit einem Material Mⱼ zu beschichten, das bestimmte Eigenschaften eines Soll-Materials Mᵢ aufweist sowie der schnellen, genauen prozessbegleitenden Qualitätskontrolle jedes Beschichtungsvorgangs. Zusätzlich können die Optimierungskriterien mit einer jeweiligen Wichtung 42a, ..., 42p gewichtet werden, um die Optimierung 14 (siehe Fig. 1 und 3) besser auf die Zielsetzung abzustimmen. So werden besonders prozessrelevante Materialeigenschaften hoch (mit einem Faktor nahe eins) und für den Prozess irrelevante Materialeigenschaften mit einem Faktor nahe Null gewichtet. Ebenso können zeit- oder kostenintensive Messverfahren S1, ..., Sm geringer gewichtet werden, so dass es unwahrscheinlicher wird, dass sie in die optimierte Auswahl 210i aufgenommen werden. Im Besonderen bezieht sich die Optimierung auf die Minimierung des Abstandes δ, von der erweitertenSignatur 100i zu der abgeleiteten Soll-Signatur 300i eines Soll-Materials Mᵢ. Dieser Abstand δ gibt den Optimierungsfehler an, mit dem man eine erweiterte Signatur 100i aus einer Soll-Signatur 200i rekonstruieren kann. Das Optimierungsergebnis kann somit ein Kompromiss aus Trennschärfe, Messkosten oder Messgeschwindigkeit sein.

**Figur 3** zeigt eine schematische Darstellung der erfindungsgemäßen Analyseverfahrensschritte zur Identifizierung bzw. Diskriminierung eines Materials aus bzw. gegen Soll-Materialien. Die "off-line"-Schrittfolge 10 umfasst die Schritte 11, 12, 13 und 14, die bereits bezüglich Figur 1 und Figur 2 beschrieben wurden. Zusätzlich werden die Optimierungsergebnisse wie die abgeleiteten Soll-Signaturen 300i für jedes Soll-Material Mᵢ im Schritt 15 in einer Soll-Datenbasis gespeichert und an eine externe Datenbasis 3 übermittelt. Darauf enthalten ist z.B. alle Information darüber, welche Soll-Materialien für die Verarbeitung in einem Produktionsprozess geeignet sind. Ferner ist eine Verfahrensvorschrift enthalten, wie ein Material Mⱼ gegen die Soll-Materialien abzugleichen ist.

Darauf wird eine "in-line" Schrittfolge 20 ausgeführt. Der Begriff "in-line" bezeichnet, dass diese Schrittfolge 20 im Gegensatz zur "off-line"-Schrittfolge 10 zur Integration in eine Produktionslinie vorgesehen ist.

Ein besonderer Vorteil des erfindungsgemäßen Analyseverfahrens und Systems ist, dass eine automatisierbare Prozesskontrolle ermöglicht ist. In einem ersten Schritt 21 wird die Auswahl 210i von l Messmethoden S1, ..., Sl auf zu verarbeitende oder zu prüfende Materialien Mⱼ, Mₕ jeweils für jedes relevante Soll-Material Mᵢ angewendet. Daraus wird eine Ist-Signatur 200j bzw. 200h bezogen auf ein Sollmaterial Mᵢ erzeugt. Im Folgeschritt 22 wird die Korrelation 220i auf die Ist-Signaturen 200j bzw. 200h angewendet, um abgeleitete Ist-Signaturen 300j bzw. 300h zu erzeugen. Alternativ kann auch bei jedem Prüfschritt der Optimierungsschritt 14 ausgeführt werden. Im abschließenden Schritt 24 erfolgt die Identifikation bzw. Diskriminierung der Materialien Mⱼ, Mₕ von dem Soll-Material Mᵢ bzw. deren Eignungsprüfung zur Verarbeitung. Dazu wird bevorzugt die Vektornorm aus der Differenz von der abgeleiteten Soll-Signatur 300i mit der jeweiligen abgeleiteten Ist-Signatur 300j bzw. 300h gebildet. Dabei wird die abgeleitete Soll-Signatur 300i und/oder die Korrelation 220i aus der externen Datenbasis 3 bezogen. Diese Abweichung δij wird mit einer Toleranz Δ verglichen. Ist die Abweichung δij für ein Material Mⱼ kleiner als die Toleranz Δ, so gilt es als der durch ein Soll-Material Mᵢ definierten Materialklasse als zugehörig bzw. für einen Produktionsprozess geeignet. Ist die Abweichung δij für ein Material Mₕ größer als die Toleranz Δ, so gilt es als der durch ein Soll-Material Mᵢ definierten Materialklasse als nicht zugehörig bzw. für einen Produktionsprozess ungeeignet.

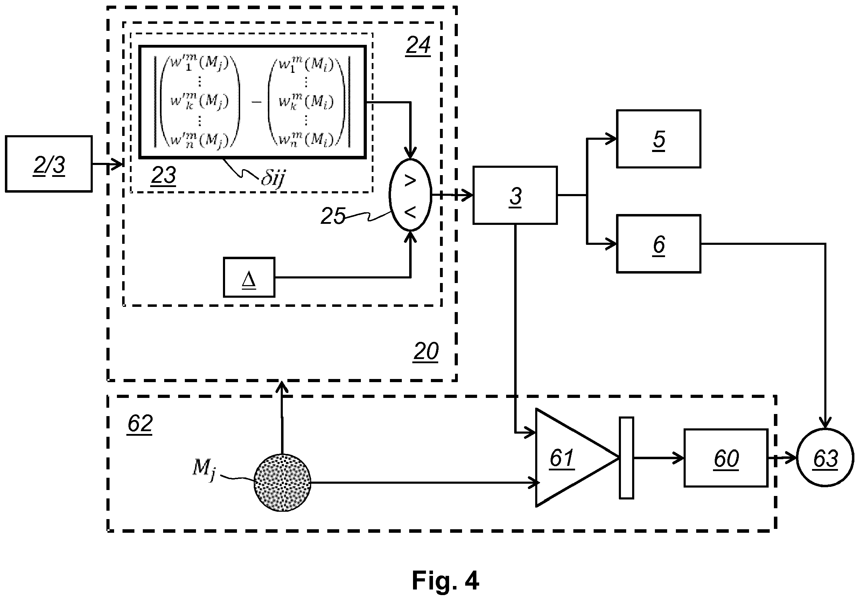

**Figur 4** gibt einen schematischen Überblick über das erfindungsgemäße Analyseverfahren. Aus der Soll-Datenbasis 2 oder der externen Datenbasis 3 werden Soll-Datensätze bezogen und die bezüglich Figur 3 beschriebene "in-line" Schrittfolge 20 durchgeführt. Der Schritt 24 wird hier detaillierter beschrieben. Nach dem Vergleichen 23 von der Ist-Signatur 300j und der Soll-Signatur 300i sowie der Normbildung daraus, folgt der Größenabgleich 25 mit der Toleranz Δ. Das Prüfergebnis und/oder die ermittelte abgeleitete oder Ist-Signatur 300j bzw. 200j wird auf der externen Datenbasis 3 dokumentiert. Diese Prüfdatensätze können anschließend an eine weitere externe Prüfdatenbasis 5 oder eine Produktdatenbasis 6 übermittelt werden. Prüfdaten aus der Produktdatenbasis 6 können beispielsweise auf einem Etikett oder einem RFID-Chip alphanumerisch, als Barcode oder als digitaler Datensatz gespeichert werden, welche auslesbar mit einem Produkt 63 verbunden werden, um das Produkt 63 eindeutig zu kennzeichnen und auszuweisen. Die weitere externe Datenbasis 5 liegt vorzugsweise im Herrschaftsbereich des Betreibers oder Herstellers der Vorrichtung 60 zur Produktbearbeitung. Die eine kontinuierliche, vorzugsweise verschlüsselte Übermittlung der Prüfdatensätze an die weitere externe Datenbasis 5 bietet die Möglichkeit zur automatisierten, nachvollziehbaren und manipulationssicheren Qualitätskontrolle. Die Toleranz Δ kann je nach Zielsetzung des erfindungsgemäßen Analyseverfahrens spezifiziert oder dynamisch angepasst werden. Wird sie dynamisch vom Bediener der Vorrichtung 60 zur Produktbearbeitung angepasst, empfiehlt sich die Speicherung ihre Zeitverlaufes auf der externen Datenbasis 3 und die Übermittlung an die weitere externe Datenbasis 5 oder die Produktdatenbasis 6.

Parallel findet das Verarbeiten 62 des Materials Mⱼ in der Vorrichtung 60 zur Produktbearbeitung zu einem Produkt 63 statt. Eine Zufuhreinrichtung 61 kann dabei die Zufuhr des Materials Mⱼ in Abhängigkeit von den Prüfdatensätzen auf der externen Datenbasis regeln.

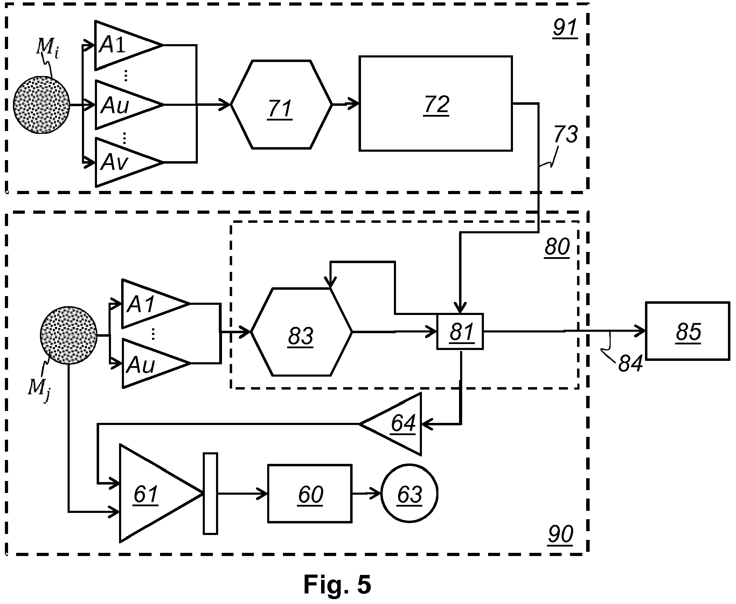

**Figur 5** zeigt eine schematische Darstellung des erfindungsgemäßen Systems 90 zur Produktbearbeitung. Für ein zur Verarbeitung vorgesehenes Material Mⱼ wird durch einen Satz von u Messapparaturen A1, ..., Au eine Signatur 200j erzeugt. Ein Datenverarbeitungssystem 80 liest die Ist-Signatur 200j von den u Messapparaturen A1, ..., Au und Soll-Datensätze von einem externen Speichermedium 81 aus. Ferner prüft eine Datenverarbeitungseinrichtung 83 des Datenverarbeitungssystems 80 das Material Mⱼ durch Ausführen der in Figur 2 beschriebenen "in-line" Schrittfolge 20. Die Prüfergebnisse werden als Prüfdatensätze auf dem externen Speichermedium 3 protokolliert und vorzugsweise verschlüsselt über eine Datenfernverbindung 84 an ein weiteres externes Speichermedium 85 übermittelt. Ferner können die Prüfdatensätze an eine Steuereinheit 64 übermittelt werden, welche in Anhängigkeit dieser Prüfdatensätze eine Zufuhreinrichtung 61 der Vorrichtung 60 zur Produktbearbeitung für das Material Mⱼ regelt. Ergibt die Prüfung beispielsweise, dass das Material Mⱼ zum Verarbeiten 62 in der Vorrichtung 60 zur Produktbearbeitung ungeeignet ist, wird seine Zufuhr gestoppt, so dass es nicht in einem Produkt 63 verarbeitet wird.

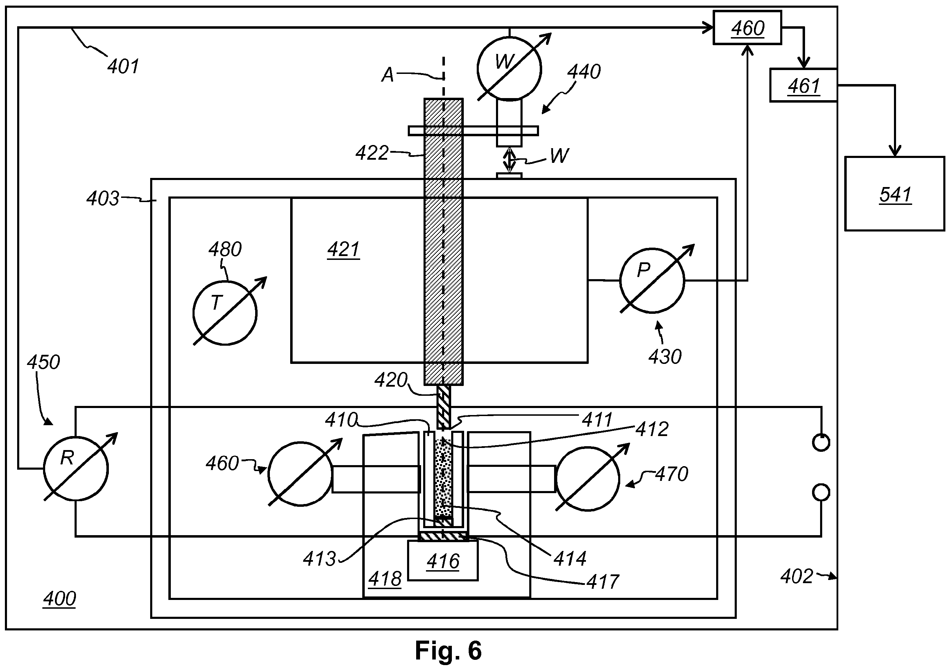

**Figur 6** zeigt eine schematische Darstellung einer Ausführungsform der Messvorrichtung 400 zur Erfassung von Materialeigenschaften von Materialien Mⱼ, die nicht Teil der Erfindung ist. Die Messvorrichtung 400 kann in einem von außen zugänglichen Gehäuse 402 aufgenommen sein. Insbesondere eignet sich diese Messvorrichtung für komprimierbare Materialen Mⱼ, wie z.B. Pulver oder Gase, und im Speziellen für elektrisch leitfähige Pulver. Es können jedoch grundsätzlich auch wenig komprimierbare Flüssigkeiten vermessen werden. Suspensionen von festen Partikeln in Flüssigkeiten können unter kontrollierten und reproduzierbaren Bedingungen zumindest teilweise getrocknet werden, so dass sie in Pulverform vorliegen.

Die Messvorrichtung 400 umfasst einen elektrisch isolierenden Prüfkörper 410 mit einem unteren elektrisch leitenden Verschluss 413, einer oberen Öffnung 411 und einem Hohlraum 412 zur Aufnahme einer Probe 414 an Material Mⱼ. Der Prüfkörper 410 ist bevorzugt zylinderförmig ausgebildet. Um vergleichbare und reproduzierbare Messergebnisse zu erhalten, werden die Anfangsbedingungen beim Abfüllen der Probe 414 in den Prüfkörper 410 in einer definierten Weise eingestellt. Beispielsweise besteht die Probe 414 aus einer definierten Gewichtsmenge an Pulver. Das Pulver kann vermittels einer Waage abgewogen werden. Vorzugsweise wird es jedoch von einer Pulverdosieranlage (nicht dargestellt) abgefüllt, die nicht nur das Gewicht, sondern auch rheologisch relevante Größen wie die Verdichtung des Pulvers oder messrelevante Größen wie die Form der Oberfläche 415 des Pulvers oder Lufteinschlüsse im Prüfkörper 410 kontrollierbar macht. Die Aussagekraft der Messungen steigt, wenn z.B. dieselbe oder eine gleichartige Pulverdosieranlage eine Vorrichtung zur Produktbearbeitung 60 beschickt. Der Prüfkörper 410 sitzt für die Messung in einem Prüfkörperhalter 418. Der Prüfkörper 410 und der Prüfkörperhalter 418 können korrespondierende Positionierelemente (nicht dargestellt) tragen, um eine eindeutige Positionierung des Prüfkörpers 410 im Prüfkörperhalter 418 zu erhalten.

Die Messvorrichtung 400 umfasst ferner mehrere dem Prüfkörper 410 zugeordnete Messapparaturen A1, ..., Au zur Ausführung von l unterschiedlichen Messmethoden S1, ..., Sl an der Probe 414 zur Erfassung einer Ist-Signatur 220j des Materials Mⱼ aus mindesten l zeitabhängigen Messwerten.

Eine der Messapparaturen A1, ..., Aubesteht aus einem elektrisch leitfähigen Druckstempel 420 und einem Druckmesser 430 zur Erfassung des vom Druckstempel 420 auf die Probe 414) beaufschlagten Drucks P. Der Druckstempel 420 ist zur Druckbeaufschlagung der Probe 414 im Hohlraum 412 des Prüfkörpers 410 formschlüssig mit der oberen Öffnung 411 und entlang einer Achse A der Messvorrichtung 400 in den Prüfkörper 410 einführbar. Der Druckstempel 420 ist über eine Stange 422 mit einem pneumatischen Motor 421 gekoppelt. Der Antriebsdruck des pneumatischen Motors 421 ist ein Maß für den Druck P, der vom Druckstempel 420 auf die Probe 414 ausgeübt wird. Aufgrund der Formschlüssigkeit und folglich Flächengleichheit von Druckstempel 420 und Öffnung 411 des Prüfkörpers 410 ist der Druck P wiederum ein direktes Maß für die auf die Probe 414 wirkende Kraft. Bei einer typischen Messung wird ein Anfangsdruck, z.B. 0 bar, beaufschlagt, der in geeigneten Intervallen, z.B. 100 mbar, bis zu einem Enddruck, z.B. 5 oder 6 bar, erhöht wird. Der Druck P wird in Abhängigkeit von der Zeit erfasst.

Eine weitere der Messapparaturen A1, ..., Au ist ein Wegmesser 440 zur Erfassung eines durch den Druckstempel 420 entlang der Achse A zurückgelegten Weges W. Beispielsweise ist hierzu ein induktiver Wegmesser geeignet, der den Weg W erfasst, den die Stange 422 relativ zu einem Gerüst 403, das den pneumatischen Motor 421 und den Prüfkörperhalter 418 starr haltert, zurücklegt. Folglich entspricht dieser Weg W dem Weg W des Druckstempels 420 entlang der Achse A. Der Weg W wird während der Druckbeaufschlagung des Druckstempels 420 in Abhängigkeit von der Zeit erfasst.

Eine weitere der Messapparaturen A1, ..., Au ist ein elektrisches Messgerät 450. Der Druckstempel 420 ist elektrisch leitfähig. Ist der Druckstempel 420 in Kontakt mit der Probe 414, so bildet sich ein Strompfad entlang der Achse A vom Druckstempel 420 durch die Probe 414 in den elektrisch leitenden Verschluss 413 des Prüfkörpers 410. Am Druckstempel 420 und am Verschluss 413 ist das elektrische Messgerät 450 zur Messung des bezogen auf die Achse A Längswiderstandes R in 2-Punkt-Messkonfiguration oder vorzugsweise in stromloser 4-Punkt- Messkonfiguration angeschlossen, wie in Figur 6 dargestellt. Denkbar ist auch die Messung eines Längswiderstandes bezüglich der Achse A oder die zusätzliche Bestimmung der Temperatur T der Probe durch ein oder mehrere Thermometer 480, das z.B. als Peltier-Element ausgebildet ist. Dadurch kann die durch die Kompression der Probe 414 bewirkte Temperaturänderung ggf. an verschiedenen Orten der Probe 414 erfasst werden und daraus Rückschlüsse auf Wärmekapazität oder Wärmeleitfähigkeit des Materials Mⱼ gezogen werden. Das elektrische Messgerät 450 umfasst beispielsweise eine Spannungsquelle und einen Widerstandsmesser, vorzugsweise mit mehreren Vorwiderständen (nicht dargestellt) und einem Relais (nicht dargestellt) zur Beschaltung der Vorwiderstände zum vorzugsweise automatisierten Umstellen zwischen verschiedenen Messbereichen für hochohmige und niederohmige Proben. Der Längswiderstand R der Probe 414 wird während der Druckbeaufschlagung des Druckstempels 420 als Funktion der Zeit erfasst. Ein anfänglich hochohmiges Pulver kann während der Druckbeaufschlagung sukzessive niederohmig werden, so dass zweckmäßigerweise der Messbereich während einer typischen Messung ein oder mehrmals umgestellt werden kann.

Eine weitere der Messapparaturen A1, ..., Au kann ein optisches Messgerät 460 sein. Das optische Messgerät 460 eine CCD-Kamera sein. Ebenso kann die CCD-Kamera mit einer Beleuchtungseinrichtung ausgestaltet sein, um ein Abbild eines Teils der Oberfläche 415 der Probe 414 unter definierten, umgebungsunabhängigen Beleuchtungsbedingungen zu erhalten. Denkbar ist auch, eine Kalibrierprobe (nicht dargestellt) im optischen Pfad zwischen Probe 414 und Kamera anzuordnen. Die Kalibrierprobe kann ein Loch umfassen, durch das die Oberfläche 415 ablichtbar ist, sowie eine Farbkalibriermarke aus mindestens drei einen Farbraum (z.B. RGB) aufspannenden Farbsegmenten, einer Weiss-Marke zur Kalibrierung der Beleuchtungsverhältnisse bzw. der Helligkeitsverteilung und/oder eine Weiss-Schwarz-Marke zur Fokussierung der Kamera und zur Kalibrierung der Kantenschärfe oder des Kontrasts. Die Kalibrierungen können vor jeder Messung und computergestützt erfolgen. Das optische Abbild der Probe 414 kann während der Druckbeaufschlagung des Druckstempels 420 in Abhängigkeit von der Zeit erfasst werden. Alternativ wird es nur einmal oder mehrmals erfasst, vorzugsweise zu definierten Zeitpunkten, z.B. wenn die Probe 414 mit dem Anfangsdruck oder mit dem Enddruck beaufschlagt worden ist, weil dann die grundsätzlich druckbedingte Morphologie des Pulvers in der Probe 414 in einem definierten und reproduzierbaren Zustand ist. Die optische Messung kann über jeden Spektralbereich erfolgen, z.B. über den sichtbaren, einen infraroten insbesondere zur Temperaturerfassung oder im Röntgenbereich zur Erfassung von Strukturen der Probe 414 unter seiner Oberfläche 415. Insbesondere kann das optische Messgerät 460 im Prüfkörperhalter 418 gehaltert und auf den Prüfkörper 410 gerichtet sein. Der Prüfkörper 410 ist dann zumindest teilflächig und/oder in den zu messenden Spektralbereichen transparent ausgestaltet.

Eine weitere der Messapparaturen A1, ..., Au kann ein aktives oder passives akustisches Messgerät 470 zur Erfassung des akustischen Eigenspektrums der Probe 414 sein. Insbesondere kann das akustische Messgerät 470 im Prüfkörperhalter 418 gehaltert und auf den Prüfkörper 410 gerichtet und über den Prüfkörper 410 mit dem Pulver akustisch gekoppelt sein. Dazu ist der Prüfkörper 410 so ausgebildet, dass er in den zu messenden akustischen Frequenzbereichen resonant ist. Akustische Messgeräte 470 können Informationen über Zustände von unbewegten oder bewegten Flüssigkeiten, Pulvern oder Schüttgütern liefern, z.B. über den Füllstand im Prüfkörper 410, Strömungsgeschwindigkeit des Pulvers oder An- oder Abwesenheit von Verunreinigungen oder Fremdkörpern. Akustische Wandler können aus akustischenSchwingungen elektronische Signale erzeugen. Bei passiven akustischen Sensoren wird ein Geräusch, das der beobachtete Vorgang selbst erzeugt (z.B. das Geräusch des Pulvers während der Kompression durch den Druckstempel 420) ausgewertet. Aktive akustische Sensoren erzeugen z.B. ein Ultraschallfeld, um das Pulver anzuregen und messen seine schallfrequenz- und/oder zeitaufgelöste Antwort. Aus diesen Schallreflexionen können z.B. die Lagen von Grenzflächen, durch Schallbeugung an Kanten oder aus Frequenzänderungen (z.B. vermittels des akustischen Doppler-Effekts) Geschwindigkeiten ermittelt werden. Messungen mit akustischen Sensoren sind für die meisten Materialien Mⱼ noninvasiv, schonend und an die jeweiligen Erfordernisse eines Materials Mⱼ flexibel anpassbar, z.B. durch Modulation der anregenden Schallfrequenz.Das akustische Messgerät 470 kann beispielsweise einen piezoelektrischen Aktuator oder Sensor umfassen.

Piezoelektrische Bauteile sind robust, zuverlässig, einfach zu handhaben, kompakt ausgestaltbar und flexibel einsetzbar. Ein piezoelektrisches Material wandelt eine anliegende elektrische Spannung in mechanische bzw. akustische Schwingungen (Aktuatorbetrieb) oder umgekehrt (Sensorbetrieb). Ein akustisch an die Probe 414 angekoppelter Piezokristall bzw. Piezokeramik kann folglich ihre mechanischen Vibrationen aufnehmen und direkt in ein elektrisches Messsignal wandeln. Das Frequenzspektrum dieses elektrischen Signals hängt auf eindeutige mit dem Frequenzspektrum der vom Piezo erfassten mechanischen Schwingungen zusammen. Die zu messende Information ist aus dem Frequenzspektrum, z.B. seinem Amplitudenspektrum und seinem Phasenspektrum, extrahierbar und besteht beispielsweise in für das Material Mⱼ typischen Resonanzfrequenzen. Vorzugsweise werden die akustischen Messungen während der Druckbeaufschlagung des Druckstempels 420 in Abhängigkeit von der Zeit erfasst.

Denkbar ist auch, dass optische Messgeräte 460, akustische Messgeräte 470 und/oder Thermometer 480 im Druckstempel 420 und/oder im Verschluss 413 des Prüfkörpers 410 integriert sind.

Die Messvorrichtung 400 umfasst schließlich eine Datenerfassungsvorrichtung 460, die mit den Messapparaturen A1, ..., Au über Datenleitungen 401 kommunikativ verbunden, so dass sie k zeitabhängige Messwerte der Ist-Signatur 200j erfassen kann. Insbesondere ist die Datenerfassungsvorrichtung 460 zur synchronisierten Erfassung dieser k zeitabhängigen Messwerte ausgelegt. Vorzugsweise ist Datenerfassungsvorrichtung 460 zu dem ein Analog-Digitalwandler, der die von den Messapparaturen A1, ..., Au meist analog ausgegebenen Messwerte in digitale Werte wandelt. Durch die Synchronisierung kann die Zeit t als Korrelationsgröße zwischen Messwerten verschiedener Messapparaturen A1, ..., Au fungieren. Beispielsweise messen der Druckmesser 430 den Druck P=P(t) und der Wegmesser 440 den Weg W=W(t) unter einander synchronisiert jeweils als Messkurve der Zeit t, so dass sich Druck P und Weg W z.B. zu einer Messkurve P(W) des Drucks P in Abhängigkeit vom Weg W korrelieren lassen. Der Informationsgehalt dieser korrelierten Messkurve P(W) ist höher als der von zeitlich unkorrelierten Messkurven des Drucks P=P(t) und des Wegs W=W(t). Der zusätzliche Informationsgehalt ist durch Fitten der Messkurve z.B. mit einer Sigmoidfunktion in Form von Fitparametern einfach zu ermitteln. Analog lassen sich höher dimensionale Messkurven erstellen und auswerten, z.B. der Druck P=P(W,R,T) in Anhängigkeit vom Weg W, dem Längswiderstand R und einer Temperatur T der Probe 414. Die Ist-Signaturen 200j oder erweiterten Soll-Signaturen 100i können sowohl zeitlich korrelierte und unkorrelierte Werte umfassen. Ein Beispiel für einen mit dem Druck P=P(W,R,T) zeitlich unkorrelierten Wert sind Werte, die aus einem einmalig aufgenommenen Bild der Probe 414 extrahiert worden sind. In den abgeleiteten Ist-Signaturen 300j und abgeleiteten Soll-Signaturen 300i sind zusätzliche, "versteckte" Korrelationen enthalten, die von einem Algorithmus ausgewertet werden. Die Datenerfassungsvorrichtung 460 ist über eine standardisierte Schnittstelle wie einem USB-Port z.B. mit einem lokalen Computer 541 verbunden. Der Computer 541 kann die zeitliche Korrelation der zeitabhängigen Messwerte P=P(t), W=W(t), R=R(t) etc. ausführen, die Fitparameter aus korrelierten Messkurven wie P(W) erheben, die Ist-Signaturen 200j zusammenstellen, abgeleitete Ist-Signaturen 300j durch Anwenden der Korrelationen 220i von Soll-Materialien Mᵢ auf die die Ist-Signaturen 200j ermitteln und ggf. den Algorithmus zur Ermittlung der Auswahlen 210i, Korrelationen 220i und abgeleiteten Soll-Signaturen 300i für Soll-Materialien Mᵢ ausführen.

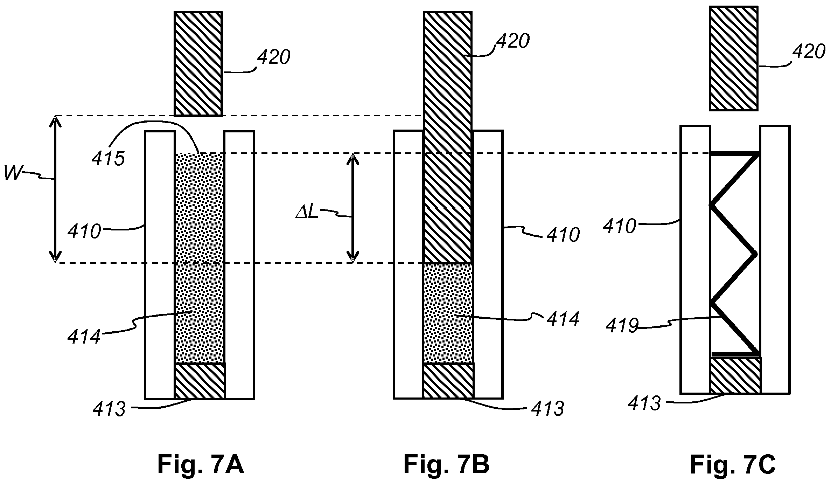

**Figuren 7A und 7B** zeigen den mit einer Probe 414 eines Materials Mⱼ befüllten Prüfkörper 410 vor bzw. nach Beaufschlagung mit einem Druck P durch den Druckstempel 420. Der Druckstempel 420 legt dabei einen Weg W zurück. Auf diesem Weg W komprimiert er die Probe 414 um einen Kompressionsweg ΔL. Der Beginn des Kompressionswegs ΔL ergibt sich z.B. aus dem Schluss des Stromkreises zwischen dem leitfähigen Druckstempel 420 über die Probe 414, den leitfähigen Verschluss 413, ggf. den Prüfkörperhalter 418 und das elektrische Messgerät 450 oder aus der Korrelation von Druck P und Weg W.

Denkbar ist ferner, die Messvorrichtung 400 regelmäßig mit einer Eichvorrichtung 419 zu kalibrieren. Die Eichvorrichtung 419 dient z.B. zur Kalibration der Messungen von Druck P, Weg W und ggf. Widerstand R und kann beispielsweise eine leitfähige Feder mit (jeweils im unkomprimierten und/oder mit einem bestimmten Druck P beaufschlagten Zustand) definiertem elektrischem Widerstand und definierter Federkonstante sein. Wie in Figur 7C gezeigt, kann die Eichvorrichtung 419 analog zu einer Probe 414 formschlüssig in einem Prüfkörper 410 der Messvorrichtung 400 umfasst sein und vermessen werden. Denkbar ist, dass ein Benutzer 511, 521, 531 der Messvorrichtung 400 die Eichvorrichtung 419 regelmäßig statt einer Probe 414 vermisst. Alternativ kann die Eichvorrichtung 419 in einem zweiten Prüfkörper 410 im Prüfkörperhalter 418 der Messvorrichtung 400 enthalten sein, so dass die Eichvorrichtung 419 gleichzeitig mit einer Probe 414 vermessen werden kann.

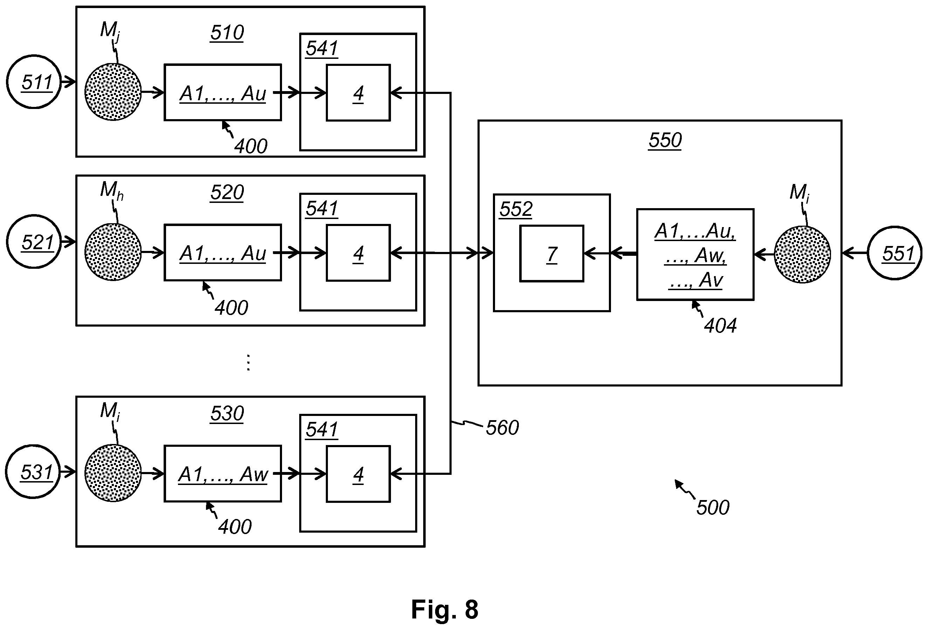

**Figur 8** zeigt eine exemplarische Ausführungsform eine eines erfindungsgemäßen Systems 500 zur Identifizierung oder Diskriminierung von Materialien Mⱼ in schematischer Darstellung. Das System 500 besteht mindestens einer lokalen Einrichtung 510, 520, 530, die je einem Benutzer 511, 521, 531 zugeordnet ist. Ferner ist mindestens eine Zentrale 550 eines Betreibers 551 dem System 500 zugeordnet.

Jede lokale Einrichtung 510, 520, 530 umfasst mindestens eine Messvorrichtung 400 zur Erfassung je mindestens einer Ist-Signatur 220j für Materialien Mⱼ. Ein Beispiel einer solchen Messvorrichtung 400, die nicht Teil der Erfindung ist, ist vorangegangen anhand der Figur 6 bis 7C beschrieben. Die Messvorrichtung 400 ist kommunikativ mit einem lokalen Computer 541 der lokalen Einrichtung 510, 520, 530 verbunden. Der lokale Computer 541 umfasst eine lokale Datenbasis 4 und dient zur Speicherung und/oder Verarbeitung der Ist-Signatur 220j.

Gemäß der erfinderischen Idee können verschiedene Typen oder Klassen von Benutzern 511, 521, 531 im System 500 integriert sein. Eine erste Klasse von Benutzern 511, 521, 531 gebraucht ihre lokale Einrichtung 510, 520, 530 zur Qualitätskontrolle von Materialien Mⱼ, Mₕ wie Pulvern für eine Plasmabeschichtungsanlage oder pulverförmiger Lebensmittel. Ihr Interesse besteht in schneller, produktionsbegleitender, kostengünstiger, zuverlässiger, dokumentierbarer und ggf. zertifizierbarer Pulveranalyse. Entsprechend umfassen die Messvorrichtungen 400 dieser ersten Klasse von Benutzern 511, 521, 531 eine Auswahl von u Messapparaturen A1, ..., Au aus allen A1, .., Au, .., Aw, ..., Av, die sich der Analyse nutzbringend auf die Materialien Mⱼ, Mₕ anwenden lassen. Welche u Messapparaturen A1, ..., Au das konkret sind, hängt von der Anwendung und den technischen Spezifikationen des jeweiligen Benutzers 511, 521, 531 ab. Diese Spezifikationen können der Zentrale 550 in Form von Metadaten übermittelt und dort ausgewertet werden, in dem sie in der Kombination 40 von Optimierungskriterien 41a, ..., 41p bzw. in ihre Wichtung 42a, ...,42p berücksichtigt werden. Beispielsweise ist die Farbzusammensetzung eines pulverförmigen Farbstoffs für eine Autolackiererei hoch und für den Hersteller von an einem Endprodukt nicht sichtbaren Grundierfarben niedrig zu gewichten.

Eine zweite Klasse von Nutzern sind beispielsweise Analyseanbieter oder Universitätslabore mit Messvorrichtungen 400, die mit Satz von w Messapparaturen A1, ..., Aw umfassen. Diese w Messapparaturen A1, ..., Aw können über die u Messapparaturen A1, ..., Au der ersten Klasse von Benutzern 511, 521, 531 hinaus oder zusätzlich Messgeräte enthalten, die aussagekräftige Messwerte auf zeit- und/oder kostenintensive Weise erheben können, z.B. eine Synchrotron oder aufwendige (nass-)chemische Verfahren. Diese zweite Klasse von Benutzern 511, 521, 531 kann Ist-Signaturen 200j zur Kontrolle nachmessen, zusätzliche Messwerte erheben, falls die Identifikation oder Diskriminierung eines Materials Mⱼ, Mₕ nicht eindeutig genug ist, oder erweiterte Soll-Signaturen 100i für Soll-Materialien Mᵢ erfassen, die noch nicht in der zentralen Datenbasis 7 gespeichert sind. Ferner können sie Prüfbedingungen, Prüfzertifikate als Metadaten übermitteln. Auch Benutzer 511, 521, 531 der ersten Klasse können gegenseitig die in den Ist-Signaturen 200j enthaltenen Messwerte überprüfen. Dieser "peer review" kann über die Zentrale 550 vermittelt werden und vorteilhafterweise gegenüber den Benutzern 511, 521, 531 anonymisiert erfolgen.

Die mindestens eine Zentrale 550 umfasst einen Server 552 mit einer zentralen Datenbank 7 zur Speicherung und/oder Verarbeitung der Ist-Signaturen 220j der lokalen Einrichtung 510, 520, 530. Auch in der Zentrale 550 kann eine oder mehrere weitere Messvorrichtungen 404 vorgesehen sein, die alle A1, ..., Au, ..., Aw, ..., Av, die sich der Analyse nutzbringend auf jedes für die Benutzer 511, 521, 531 relevantes Material Mⱼ, Mₕ anwenden lassen. Relevant können Materialien Mⱼ, Mₕ sein, die zur Verarbeitung oder Verwendung bestimmt sind, oder aber Verunreinigungen oder Giftstoffe, die bei der Verarbeitung oder Verwendung auszuschließen sind.

Ein Netzwerk 560 verbindet die lokalen Computer 541 der lokalen Einrichtung 510, 520, 530 sowie den Server kommunikativ. Diese kommunikative Verbindung kann insbesondere über das Internet oder ein Intranet einer Firma bestehen. Dieses mithin "cloudbasierte" System 500 erlaubt den Informationsaustausch unter den Benutzern 511, 521, 531. Vorzugsweise sind die lokalen Computer 541 über den Server 552 der Zentrale 550 verbunden, damit möglichst alle von den Benutzern 511, 521, 531 erhobenen Ist-Signaturen 200j für Materialien Mⱼ und ggf. erweiterten Soll-Signaturen 100i für Materialien Mⱼ in der zentralen Datenbank 7 gesammelt und vom Server 552, wie z.B. in der anhand der Figuren 1 bis 4 beschriebenen Weise, auswertbar sind. Durch diese Pflege und dieses Training der zentralen Datenbank 7 wird die Datenlage über Soll-Material Mᵢ stetig verbessert und folglich die Identifikation oder Diskriminierung der Materialien Mⱼ, Mₕ immer trennschärfer. Gleichzeitig werden die Identifikation oder Diskriminierung effizienter, weil die für einen jeweiligen Benutzer 511, 521, 531 hinreichende Genauigkeit aufgrund der verbesserten Datenlage in der zentralen Datenbank 7 immer weniger Messapparaturen A1, ..., Au bzw. Messmethoden S1, ..., Sl in der Messvorrichtung 400 der lokalen Einrichtung 510, 520, 530 erfordert. Welche Messapparaturen A1, ..., Au entbehrlich sind, wird erfindungsgemäß durch einen Algorithmus ermittelt. In der gebündelten Erfassung und Auswertung der Informationen des ganzen Systems 500 auf dem Server 552 der Zentrale 550 besteht folglich ein Mehrwert für die Benutzer 511, 521, 531 der lokalen Einrichtungen 510, 520, 530. Der Mehrwert besteht insbesondere in einer statistisch breiteren Datenbasis des Kollektivs der am System 500 beteiligten Benutzer 511, 521, 531. Dem unterliegt die Grundidee der Erfindung, die Identifikation oder Diskriminierung von komplexen Materialien Mⱼ durch ein netzwerkbasiertes Analyseverfahren durch eine gute statistische Datenbasis zu verbessern, ohne dass ein analytisches Verständnis des eventuell hochkomplex zusammengesetzten Materials Mⱼ erforderlich wäre. Ein zusätzlicher Mehrwert besteht darin, dass jeder Benutzer 511, 521, 531 auch auf den heuristischen Erfahrungsschatz der anderen Benutzer 511, 521, 531 zurückgreifen kann. Dieser Zugriff erfolgt mittelbar über den Betreiber 551 der Zentrale 550 des Systems. Die von den Benutzern 511, 521, 531 übermittelten Ist-Signaturen 200j und Metadaten sind prinzipiell sensibel, weil sie Rückschlüsse auf Betriebsgeheimnisse und Produktion zulassen. Die gegenüber Dritten anonymisierbare Bündelung dieser Daten durch einen vertrauenswürdigen Betreiber 551 der Zentrale 550 lässt alle Benutzer 511, 521, 531 ist vom gesamten Inhalt der zentralen Datenbasis 7 profitieren, ohne sensible Information preiszugeben. Denkbar ist auch, dass analog die Benutzer 511, 521, 531 ihre lokalen Datenbanken 4 pflegen und trainieren.

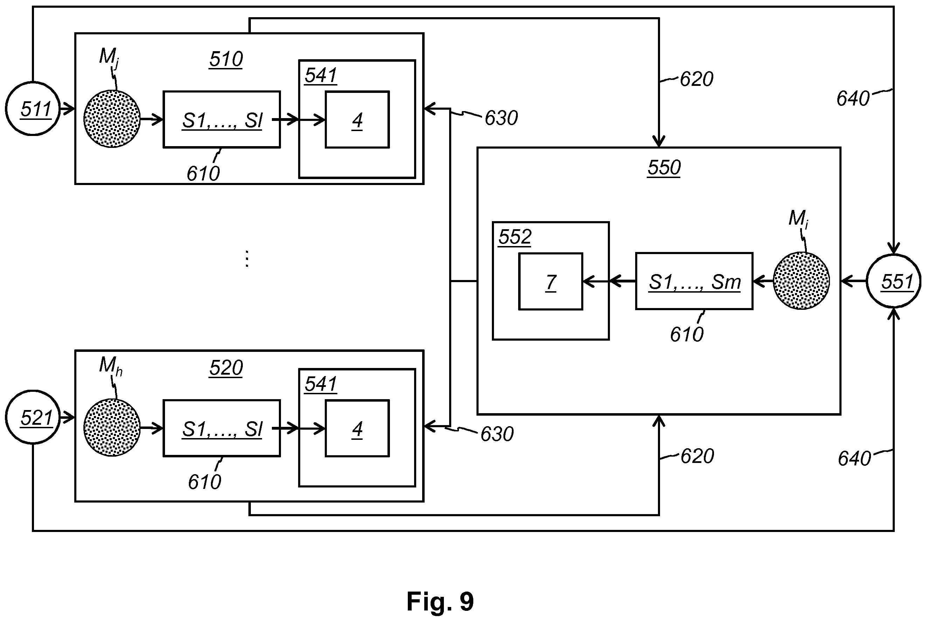

**Figur 9** zeigt eine schematische Darstellung des Verfahrens zum Betrieb eines Systems 500 zur Identifizierung oder Diskriminierung von Materialien Mⱼ, insbesondere eines wie in Figur 8 dargestellten Systems 500.

In einem ersten Schritt des Verfahrens erfolgt das Erheben 610 einer Ist-Signatur 220j aus k unterschiedlichen Messwerten an einem zu bestimmenden Material Mⱼ durch mindestens einen Benutzer 511, 521, 531 einer lokalen Einrichtung 510, 520, 530 des Systems 500. Die k unterschiedlichen Messwerte werden mit l unterschiedlichen Messmethoden S1, ..., Sl vermittels von u Messapparaturen A1, ..., Au erhoben. Die Zahl k ist größer gleich l und l ist größer gleich u, weil eine Messmethode S1, ..., Sl nur eine oder mehrere Messwerte erheben und desgleichen eine Messapparatur A1, ..., Au nur eine oder mehrere Messmethoden S1, ..., Sl ausführen kann. Selbiges gilt für die n unterschiedlichen Messwerte, die durch die insgesamt unterschiedlichen m S1, ..., Sk, ..., Sm Messmethoden vermittels der v Messapparaturen A1, ..., Av erfasst werden. Es folgt, dass k, l, u kleiner gleich n, m, v respektive sind.

Darauf folgt eine Anfrage 620 des mindestens einen Benutzers 511, 521, 531 an einen Betreiber 551 einer Zentrale 550 des Systems 500. Bei der Anfrage wird zumindest die Ist-Signatur 220j und ggf. auf den Benutzer 511, 521, 531 bezogene Metadaten übermittelt. Metadaten können z.B. die Identität des Benutzer 511, 521, 531, seine Spezifikationen für ein Material Mⱼ, seine Kommentare oder die Gerätekennung seiner Messapparaturen A1, ..., Au umfassen.

Die vom mindestens einen Benutzer 511, 521, 531 übermittelten Ist-Signaturen 220j und ggf. Metadaten werden auf dem Server 552 der Zentrale 550 des Systems direkt nicht-flüchtig gespeichert oder zwischengespeichert, ausgewertet und in ausgewerteter Form nicht-flüchtig gespeichert. Aus den Ist-Signaturen 220j und ggf. Metadaten werden je eine abgeleitete Soll-Signatur 300j erzeugt.Wie bezüglich der Figuren 1 bis 3 beschrieben, erfolgt dies durch Anwenden je einer Korrelation 200i auf eine Ist-Signatur 220j für mindestens ein Soll-Material Mᵢ. Die Korrelation 200i ist auf eine erweiterte Signatur 100i eines Soll-Materials Mi bezogen. Die erweiterten Signaturen 100i bestehen je aus n unterschiedlichen Messwerten. Auf dem Server 552 der Zentrale 550 des Systems 500 wird je einer Abweichung δij der abgeleiteten Ist-Signatur 300j für das Material Mⱼ bezogen auf die jeweilige erweiterte Signatur 100i des mindestens einen Soll-Materials Mᵢ berechnet. Anschließend erfolgt das Rückmelden 630 durch denServer 552 bzw. den Betreiber 551 der Zentrale 550 des Systems 500 an den anfragenden Benutzer 511, 521, 531, für welche Soll-Materialien Mᵢ die jeweilige Abweichung δij eines Materials Mⱼ bezogen auf ein Soll-Materials Mᵢ kleiner als eine für das Soll-Material Mᵢ festgelegte Toleranz Δ ist. Insbesondere kann dieses Ergebnis in Form einer Liste zurückgemeldet werden, die in Abhängigkeit der Differenz von Abweichung δij und Toleranz Δ sowie ggf. unter Berücksichtigung von Metadaten die Wahrscheinlichkeit in Prozent beziffert wird, mit der ein Material Mⱼ mit einem Soll-Materials Mᵢ übereinstimmt. Ist das Ergebnis uneindeutig, weil auf dieser Liste mehrere Soll-Materialien Mᵢ mit hohen Wahrscheinlichkeiten mit dem Material Mⱼ übereinstimmen, so bestehen prinzipiell zwei Möglichkeiten: Erstens kann der anfragende Benutzer 511, 521, 531 das Ergebnis z.B. anhand noch unkommunizierter Metadaten oder seiner Erfahrung bewerten, indem er unplausible Übereinstimmungen verwirft. Beispielsweise würde ein Milchpulverhersteller eine Übereinstimmung seines Milchpulvers mit einem pulverförmigen Autolack verwerfen können, selbst wenn die messtechnische Analyse eine hohe Übereinstimmung ergibt. Erfindungsgemäß kann dieses Bewerten 640 des anfragenden Benutzers 511, 521, 531 als Feedback an den Betreiber 550 des Systems rückgemeldet werden.

Zweitens besteht die Möglichkeit, dass von anfragenden Benutzern oder einem anderen Benutzer 511, 521, 531 oder vom Betreiber 551 die Messwerte der Ist-Signatur 200j zur Kontrolle erneut erhoben werden oder um weitere Messwerte bisher nicht verwandter Messmethoden S1, ..., Sm ergänzt werden. Insbesondere kann das Bewerten 640 des anfragenden Benutzers 511, 521, 531 zum weiteren Training der zentralen Datenbasis 7 und/oder als Entscheidungsgrundlage dienen, welche Messwerte nachzumessen oder zusätzlich zu erheben sind oder, ob ein neues Soll-Material Mᵢzu definieren ist. Auch können Erfahrungswerte als Metadaten gespeichert werden und bestimmten Übereinstimmungen zugeordnet werden (wie z.B. der o.g. unplausiblen Übereinstimmung zwischen Milchpulver und Autolack).

Der gesamten Erfindung unterliegt der Grundgedanke, die Analytik komplexer Materialien mit vernetzten und standarisierten Messvorrichtungen und -verfahren zu verbessern, indem eine breite statistische Datenbasis erfasst und ausgewertet wird, ohne dass ein genaues analytisches Verständnis der Zusammenhänge verschiedener Eigenschaften der komplexen Materialien erforderlich ist. Deshalb sind Merkmale, die bezüglich eines Aspektes der Erfindung, wie sie in den unabhängigen Ansprüchen definiert sind, auch auf ihre anderen Aspekte anwendbar. Einem Fachmann ist ferner offensichtlich, dass die offenbarten Merkmale auch herausgelöst aus dem zum Zwecke der Erläuterung der Erfindung lediglich exemplarischen Kontext mit den Gegenständen der unabhängigen Ansprüche kombinierbar sind.

### Bezugszeichenliste:

- 1: Messdatenbasis
- 2: Soll-Datenbasis
- 4: lokale Datenbasis
- 3: externe Datenbasis
- 5: weitere externe Datenbasis
- 6: Produktdatenbasis
- 7: zentrale Datenbasis
- 10: offline-Verfahren
- 11: Anwenden
- 12: Erstellen
- 13: Optimierungsiteration
- 14: Optimieren
- 15: Erstellen
- 20: inline-Verfahren
- 21: Messen
- 22: Erzeugen
- 23: Größenabgleich
- 24: Ermitteln
- 25: Vergleichsmethode
- 40: Kombination
- 41a: Erstes Optimierungskriterium
- 41p: p-tes Optimierungskriterium
- 42a: Wichtung des ersten Optimierungskriteriums
- 42p: Wichtung des p-ten Optimierungskriteriums
- 50: Optimierungsmethode
- 60: Vorrichtung zur Produktbearbeitung
- 61: Zufuhreinrichtung
- 62: Verarbeiten
- 63: Produkt
- 64: Steuereinheit
- 71: Computer
- 72: Speichermedium
- 73: Datenverbindung
- 80: Datenverarbeitungssystem
- 81: externes Speichermedium
- 83: Datenverarbeitungseinrichtung
- 84: Datenfernverbindung
- 85: weiteres externes Speichermedium
- 90: System
- 91: externes System
- 100i: erweiterte Signatur
- 200i: Soll-Signatur
- 200ii: i-te Iteration der modifizierten Soll-Signatur
- 200j: Ist-Signatur
- 210i: Auswahl
- 210ii: i-te Iteration der Auswahl
- 220i: Korrelation
- 220ii: i-te Iteration der Korrelation
- 300i: abgeleitete Soll-Signatur
- 300ii: i-te Iteration der abgeleiteten Soll-Signatur
- 300j: abgeleitete Ist-Signatur
- 301: Zweite modifizierte Signatur
- 400: Messvorrichtung
- 401: Datenleitungen
- 402: Gehäuse
- 403: Gerüst
- 404: weitere Messvorrichtung
- 410: Prüfkörper
- 411: obere Öffnung
- 412: Hohlraum
- 413: Verschluss
- 414: Probe
- 415: Oberfläche
- 416: Waage
- 417: elektrischer Kontakt
- 418: Prüfkörperhalter
- 419: Eichvorrichtung
- 420: Druckstempel
- 421: pneumatischer Motor
- 422: Stange
- 430: Druckmesser
- 440: Wegmesser
- 450: elektrisches Messgerät
- 451: Spannungsquelle

- 460: optisches Messgerät
- 470: akustisches Messgerät
- 480: Thermometer
- 490: Hygrometer
- 500: System
- 510, 520, 530: lokales Einrichtungen
- 511, 521, 531: Benutzer
- 541: Computer
- 550: zentrales Subsystem
- 551: Betreiber
- 552: Server
- 560: Netzwerk
- 610: Erheben
- 620: Anfrage
- 630: Rückmelden
- 640: Bewerten
- A: Achse
- A1: Erste Messapparatur
- Au: u-te Messapparatur
- Aw: w-te Messapparatur
- Av: v-te Messapparatur
- δ: Abstand
- δij: Abweichung
- Δ: Toleranz
- Mᵢ: Soll-Material
- Mⱼ: Material
- Mₕ: weiteres Material
- S1: Erste Messmethode
- Sl: l-te Messmethode
- Sm: m-te Messmethode
- ΔL: Kompressionsweg
- P: Druck
- R: Widerstand
- T: Temperatur
- W: Weg

## Patentansprüche

1. Verfahren zur Identifizierung oder Diskriminierung von Materialien (Mⱼ) für einen Produktionsprozess, das Verfahren durchgeführt mit einem System (500) und umfassend folgende Schritte:
• Erheben (610) einer Ist-Signatur (200j) an einem zu bestimmenden Material (Mⱼ) durch mindestens einen Benutzer (511, 521, 531) einer lokalen Einrichtung (510, 520, 530) des Systems (500), wobei eine Ist-Signatur (200j) ein Vektor aus k unterschiedlichen Messwerten ist, die durch Durchführung l unterschiedlicher Messmethoden (S1,...,Sl) an dem zu bestimmenden Material (Mⱼ) erhalten werden;
• Anfrage (620) des mindestens einen Benutzers (511, 521, 531) an einen Betreiber (551) einer Zentrale (550) des Systems (500), wobei mit der Anfrage die Ist-Signatur (200j) übermittelt wird;
• Nicht-flüchtiges Speichern der vom mindestens einen Benutzer (511, 521, 531) übermittelten Ist-Signatur (200j) auf einem Server (552) der Zentrale (550);
• Erzeugen je einer abgeleiteten Ist-Signatur (300j) durch Anwenden je einer durch eine Matrix gegebenen Korrelation (220i) für mindestens ein Soll-Material (Mᵢ) auf die Ist-Signatur (200j), wobei die Korrelation (220i) durch derartige Optimierung erhalten wird, dass eine Abweichung (δ) zwischen einer erweiterten Soll-Signatur (100i) des jeweiligen Soll-Materials (Mᵢ) und einer abgeleiteten Soll-Signatur (300i) des jeweiligen Soll-Materials (Mᵢ) minimiert wird, wobei die erweiterte Soll-Signatur (100i) aus n unterschiedlichen Messwerten für das Soll-Material (Mᵢ) besteht, die mit m unterschiedlichen Messmethoden (S1,...,Sm) erhalten wurden, und die Zahl n größer gleich der Zahl k ist und die Zahl m größer gleich der Zahl l ist, wobei die abgeleitete Soll-Signatur (300i) durch Anwenden der Korrelation (220i) auf eine Soll-Signatur (200i) für das jeweilige Soll-Material (Mᵢ) erhalten wird, und wobei die Soll-Signatur (200i) für das jeweilige Soll-Material (Mᵢ) ein Vektor aus k Messwerten für das Soll-Material (Mᵢ) ist, die durch Durchführung der l unterschiedlichen Messmethoden an dem Soll-Material (Mᵢ) erhalten wurden;
• Berechnen je einer Abweichung (δij) der abgeleiteten Ist-Signatur (300j) für das Material (Mⱼ) von der abgeleiteten Soll-Signatur (300i) des mindestens einen Soll-Materials (Mᵢ);
• Rückmelden (630) an den anfragenden Benutzer (511, 521, 531), für welche Soll-Materialien (Mᵢ) die jeweilige Abweichung (δij) eines Materials (Mⱼ) bezogen auf ein Soll-Material (Mᵢ) kleiner als eine für das Soll-Material (Mᵢ) festgelegte Toleranz (Δ) ist.

2. Verfahren nach Anspruch 1, wobei die Ist-Signaturen (200j) von mindestens einem Benutzer (511, 521, 531) und/oder vom Betreiber (551) mit auf den anfragenden Benutzer (511, 521, 531) und/oder auf den Betreiber (551) bezogenen Metadaten ergänzt werden.

3. Verfahren nach Anspruch 2, wobei der anfragende Benutzer (511, 521, 531) auf das Rückmelden (630) des Betreibers (551) die korrekte Identifizierung oder Diskriminierung des Materials (Mⱼ) bewertet und das Ergebnis seines Bewertens (640) als Metadaten an den Betreiber (551) rückmeldet.

4. Verfahren nach einem der Ansprüche 2 bis 3, wobei für jeden Benutzer (511, 521, 531) für mindestens ein Soll-Material (Mᵢ) die zugehörige Korrelation (220i) und die zugehörige abgeleitete Soll-Signatur (300i) nach einer Kombination (40) von Optimierungskriterien (41a, ..., 41p) mit zugehörigen Wichtungen (42a, ..., 42p) optimiert wird, wobei die Wichtungen (42a, ..., 42p) anhand von Metadaten modifiziert werden.

5. Verfahren nach Anspruch 3, wobei der Betreiber (551) und/oder ein Benutzer (511, 521, 531) anhand der Metadaten und/oder der Ist-Signaturen (200j)
• die Ist-Signaturen (200j) um durch zusätzliche Messmethoden (A1,..., Av) erfasste Messwerte erweitert und die abgeleiteten Soll-Signaturen (300i) und die Korrelationen (220i) für mindestens ein Soll-Material (Mᵢ) neu optimiert; oder
• das Material (Mⱼ) in einer lokalen Datenbank (4) und/oder in der zentralen Datenbank (7) als neues Soll-Material (Mᵢ) anlegt.

6. Verfahren nach Anspruch 1, wobei vor dem Erheben (610) der Ist-Signatur (200j) ein Auswählen (210) von l Messmethoden aus m unterschiedlichen Messmethoden (S1, ..., Sm), wobei die Zahl l kleiner gleich m ist, durchgeführt wird; danach erfolgt das Messen (21) einer Ist-Signatur (200j) des Materials (Mⱼ) mittels einer Messvorrichtung (400) zum Ausführen der l unterschiedlichen Messmethoden (S1, ..., Sl); danach ein Erzeugen (22) je einer abgeleiteten Ist-Signatur (300j) aus der gemessenen Ist-Signatur (200j) des Materials (Mⱼ) durch Anwenden mindestens einer jeweils einem Soll-Material (Mᵢ) zugeordneten Korrelation (220i) mindestens eines Soll-Materials; danach ein Ermitteln (23) je einer Abweichung (δij) der abgeleiteten Ist-Signaturen (300j) des Materials (Mⱼ) von der jeweiligen abgeleiteten Soll-Signatur (300i) des mindestens einen Soll-Materials (Mᵢ); schließlich erfolgt ein Vergleichen (24) jeder Abweichung (δij) mit einer für das jeweilige Soll-Material (Mᵢ) spezifizierten Toleranz (Δ); und letztendlich ein Ausgeben (62), für welche Soll-Materialien (Mᵢ) die jeweilige Abweichung (δij) zwischen Soll-Material (Mᵢ) und Material (Mⱼ) kleiner als die Toleranz (Δ) des jeweiligen Soll-Materials (Mᵢ) ist.

7. Verfahren nach Anspruch 6, wobei eine Soll-Datenbasis (2) zum nicht-flüchtigen Speichern von Soll-Datensätzen umfassend die abgeleiteten Soll-Signaturen (300i), die Auswahlen (210i) und die Korrelationen (220i) für das mindestens eine Soll-Material (Mᵢ) mit folgenden Schritten erzeugt wird:
• Anwenden (11) der m unterschiedlichen Messmethoden (S1, ..., Sm) zum Erzeugen von je einer erweiterten Soll-Signatur (100i) aus mindestens n Messwerten für mindestens ein Soll-Material (Mᵢ);
• Erstellen (12) einer Messdatenbasis (1) von erweiterten Soll-Signaturen (100i);
• Optimieren (14) mit einem computergestützten Algorithmus je einer abgeleiteten Soll-Signatur (300i), die durch eine zugehörige Korrelation (220i) aus einer zugehörigen Soll-Signatur (200i) erzeugt wird, welche durch eine Auswahl (210i) aus den Messmethoden (S1, ..., Sm) erzeugt wird, wobei das Optimieren (14) nach einer Kombination (40) von Optimierungskriterien (41a, ..., 41p) erfolgt, welche gemäß ihrer Relevanz für das Verarbeiten (62) eines Materials (Mⱼ) in einer Vorrichtung (60) zur Produktbearbeitung mit zugehörigen Wichtungen (42a, ..., 42p) versehen werden.;
• Erstellen (15) der Soll-Datenbasis (2) aus den abgeleiteten Soll-Signaturen (300i), Auswahlen (210i) und Korrelationen (220i).

8. Verfahren nach einem der Ansprüche 6 bis 7, wobei in einer Datenbasis (3) eine ausgewählte Untermenge von Soll-Datensätzen, welche jeweils aus der Auswahl (210i) der Messmethoden (S1, ..., Sn), der Soll-Signatur (200i), der Korrelation (220i) und/oder der abgeleiteten Soll-Signatur (300i) mindestens eines Soll-Materials (Mᵢ) bestehen, nicht-flüchtig gespeichert wird, und/oder wobei in der Datenbasis (3) während des Verarbeitens (62) Prüfdatensätze, welche aus den Ist-Signaturen (200j), den abgeleiteten Ist-Signaturen (300j) und/oder den Abweichungen (δij) bestehen, protokolliert werden.

9. Verfahren nach Anspruch 8, wobei die Prüfdatensätze der Datenbasis (3) automatisch über ein digitales Netzwerk an eine weitere, externe Datenbasis (5) übermittelt werden und wobei die Prüfdatensätze auf der Datenbasis (3) als Steuerparameter für einen Regelkreis beim Verarbeiten (62) in einer Vorrichtung (60) zur Produktbearbeitung verwendet werden.

10. Verfahren nach Anspruch 9, wobei ein externes Speichermedium (81), auf dem Soll-Datensätze und/oder Prüfdatensätze gespeichert sind, mit einem Gebinde, einer Verpackungen und/oder einer Charge eines Soll-Materials (Mᵢ) oder eines Materials (Mⱼ) auslesbar verbunden wird, und wobei das externe Speichermedium (81) aus einem Radio-Frequency Identification (RFID)-Chip besteht, auf dem Datensätze in Form von RFID-Tags gespeichert werden und/oder wobei das Speichermedium (81) aus einem Etikett besteht, auf dem Datensätze alphanumerisch und/oder als Barcode gespeichert werden.

11. Verfahren nach einem der Ansprüche 6 bis 10, wobei die Eignung des mindestens einen Materials (Mⱼ) für das Verarbeiten (62) in der Vorrichtung (60) zur Produktbearbeitung während des laufenden Produktionsprozesses überprüft wird.

12. Verfahren nach einem der Ansprüche 6 bis 11, wobei das Anwenden der Auswahl (210i) der l Messmethoden (S1, ..., Sl) mindestens zehnmal schneller ist als das Anwenden (11) der m Messmethoden (S1, ..., Sm).

13. System (500) ausgebildet zur Durchführung eines Verfahrens zur Identifizierung oder Diskriminierung von Materialien (Mⱼ) gemäß einem der Ansprüche 1 bis 12, **gekennzeichnet durch**:
• mindestens eine lokale Einrichtung (510, 520, 530) jeweils umfassend mindestens eine Messvorrichtung (400) zur Erfassung je mindestens einer Ist-Signatur (200j) für Materialien (Mⱼ) und mindestens einen mit der mindestens einen Messvorrichtung (400) kommunikativ verbundenen lokalen Computer (541) mit einer lokalen Datenbasis (4) zur Speicherung und/oder Verarbeitung der Ist-Signatur (200j);
• mindestens eine Zentrale (550) umfassend einen Server (552) mit einer zentralen Datenbank (7) zur Speicherung und/oder Verarbeitung der Ist-Signaturen (200j) der mindestens einen lokalen Einrichtung (510, 520, 530); und
• ein Netzwerk (560), das den mindestens einen lokalen Computer (541) der mindestens einen lokalen Einrichtung (510, 520, 530) und den Server (552) der Zentrale (550) kommunikativ verbindet.

14. System (500) nach Anspruch 13, wobei die mindestens eine Messvorrichtung (400) einer lokalen Einrichtung (510, 520, 530) umfasst:
• mehrere Messapparaturen (A1, ..., Au) zur Ausführung von l unterschiedlichen Messmethoden (S1, ..., Sl) an zumindest einer Probe (414) eines Materials (Mⱼ) zur Erfassung einer Ist-Signatur (200j) aus k zeitabhängigen Messwerten erzeugt von v Messapparaturen (A1, ..., Av) zur Ausführung von m unterschiedlichen Messmethoden (S1, ..., Sm) zum Bestimmen je einer erweiterten Soll-Signatur (100i) aus n Messwerten für mindestens ein Soll-Material (Mᵢ), wobei die Zahlen u, l und k kleiner gleich v, m und n respektive sind; und
• eine Datenerfassungsvorrichtung (460), die mit den u Messapparaturen (A1, ..., Au) zur synchronisierten Erfassung der k zeitabhängigen Messwerte der Ist-Signatur (200j) und dem mindestens einen lokalen Computer (541) elektrisch verbunden ist.

15. System (500) nach Anspruch 13 oder 14, wobei das System (500) alle Messapparaturen (A1, ..., Av) zur Ausführung aller m unterschiedlicher Messmethoden (S1, ..., Sm) zum Bestimmen je einer erweiterten Soll-Signatur (100i) aus n Messwerten für mindestens ein Soll-Material (Mᵢ) umfasst, so dass die erweiterten Soll-Signaturen (100i) durch das System (500) erhebbar und nicht-flüchtig in der zentralen Datenbank (7) speicherbar sind.

## Claims

1. A method for identifying or distinguishing materials (Mⱼ) for a production process, the method being carried out with a system (500) and comprising the following steps:
recording (610) of an actual signature (200j) on a material to be identified (Mⱼ) by at least one user (511, 521, 531) of a local facility (510, 520, 530) of the system (500), wherein an actual signature (200j) is a vector of k different measurement values, which are obtained by carrying out I different measurement methods (S1, ..., SI) on the material (Mⱼ) to be identified;
querying (620) of the at least one user (511, 521, 531) to an operator (551) of a central station (550) of the system (500), wherein the actual signature (200j) is transmitted with the query;
non-volatile storing of the actual signature (200j) transmitted by the at least one user (511, 521, 531) on a server (552) of the central station (550);
generating one derived actual signature (300j) each by applying one correlation (220i) each, given by a matrix, for at least one target material (Mi) with regard to the actual signature (200j), wherein the correlation (220i) is obtained by optimization in such a way that a deviation (δ) between an enhanced target signature (100i) of the respective target material (Mi) and a derived target signature (300i) of the respective target material (Mi) is minimized, wherein the enhanced target signature (100i) consists of n different measurement values for the target material (Mᵢ), which measurement values were obtained with m different measurement methods (S1, ..., Sm), and the number n is greater than or equal to the number k and the number m is greater than or equal to the number I, wherein the derived target signature (300i) is obtained by applying the correlation (220i) to a target signature (200i) for the respective target material (Mᵢ), and wherein the target signature (200i) for the respective target material (Mᵢ) is a vector of k measurement values for the target material (Mᵢ), which measurement values were obtained by carrying out the I different measurement methods on the target material (Mᵢ);
calculating one deviation (δij) of the derived actual signature (300j) each for the material (Mⱼ) from the derived target signature (300i) of the at least one target material (Mᵢ);
providing feedback (630) to the querying user (511, 521, 531) regarding the target materials (Mᵢ) for which the respective deviation (δij) of a material (Mⱼ) with respect to a target material (Mᵢ) is less than a tolerance (Δ) defined for the target material (Mᵢ).

2. The method according to claim 1, wherein the actual signatures (200j) are supplemented by at least one user (511, 521, 531) and/or by the operator (551) with metadata relating to the querying user (511, 521, 531) and/or to the operator (551).

3. The method according to claim 2, wherein the querying user (511, 521, 531), in response to the feedback (630) of the operator (551), evaluates the correct identification or distinction of the material (Mⱼ) and reports the result of his evaluation (640) back to the operator (551) as metadata.

4. The method according to any one of claims 2 to 3, wherein for each user (511, 521, 531) and for at least one target material (Mᵢ), the corresponding correlation (220i) and the corresponding derived target signature (300i) are optimized after a combination (40) of optimization criteria (41a, ..., 41p) with corresponding weightings (42a, ..., 42p), wherein the weightings (42a, ..., 42p) are modified on the basis of metadata.

5. The method according to claim 3, wherein the operator (551) and/or a user (511, 521, 531), on the basis of the metadata and/or the actual signatures (200j), carry out the following steps:
enhancing the actual signatures (200j) by measurement values recorded by additional measurement methods (A1, ..., Av) and re-optimizing the derived target signatures (300i) and the correlations (220i) for at least one target material (Mᵢ); or
storing the material (Mⱼ) in a local database (4) and/or in the central database (7) as a new target material (Mᵢ).

6. The method according to claim 1, wherein prior to recording (610) the actual signature (200j), I measurement methods are selected (210) from m different measurement methods (S1, ..., Sm), the number I being less than or equal to m; an actual signature (200j) of the material (Mⱼ) is then measured (21) by means of a measuring device (400) for carrying out the I different measurement methods (S1, ..., Sl); one derived actual signature (300j) each is then generated (22) from the measured actual signature (200j) of the material (Mⱼ) by applying at least one correlation (220i) of at least one target material (Mᵢ) corresponding to one target material in each case; one deviation (δij) of the derived actual signatures (300j) of the material (Mⱼ) from the respective derived target signature (300i) of the at least one target material (Mᵢ) is then determined (23) in each case; each deviation (δij) is subsequently compared (24) to a tolerance (Δ) specified for the respective target material (Mᵢ); and lastly an output (62) regarding the target materials (Mᵢ) for which the respective deviation (δij) between a target material (Mᵢ) and a material (Mⱼ) is less than the tolerance (Δ) of the respective target material (Mᵢ).

7. The method according to claim 6, wherein a target database (2) for the non-volatile storage of target datasets comprising the derived target signatures (300i), the selections (210i) and the correlations (220i) for the at least one target material (Mᵢ) is created by means of the following steps:
applying (11) of the m different measurement methods (S1, ..., Sm) for generating one enhanced target signature (100i) each from at least n measurement values for at least one target material (Mᵢ);
creating (12) of a measurement database (1) of enhanced target signatures (100i);
optimizing (14), with a computer-based algorithm, of one derived target signature (300i) each, which is generated by a corresponding correlation (220i) from a corresponding target signature (220i), which is generated by a selection (210i) from the measurement methods (S1, ..., Sm), wherein the optimization (14) is performed after a combination (40) of optimization criteria (41a, ..., 41p), which are provided with corresponding weightings (42a, ..., 42p) according to their relevance for the processing (62) of a material (Mⱼ) in a device (60) for product processing;
creating (15) of the target database (2) from the derived target signatures (300i), selections (210i) and correlations (220i).

8. The method according to any one of claims 6 to 7, wherein a selected subset of target datasets, each consisting of the selection (210i) of measurement methods (S1, ..., Sn), the target signature (200i), the correlation (220i) and/or the derived target signature (300i) of at least one target material (Mᵢ), are stored in a non-volatile manner in a database (3), and/or wherein audit datasets consisting of the actual signatures (200j), the derived actual signatures (300j) and/or the deviations (δij) are logged in the database (3) during the processing (62).

9. The method according to claim 8, wherein the audit datasets of the database (3) are automatically transmitted via a digital network to a further, external database (5), and wherein the audit datasets are used on the database (3) as control parameters for a control circuit during the processing (62) in a device (60) for product processing.

10. The method according to claim 9, wherein an external storage medium (81) on which target datasets and/or audit datasets are stored is readably connected to a container, a pack and/or a batch of a target material (Mᵢ) or material (Mⱼ), and wherein the external storage medium (81) consists of a radio frequency identification (RFID) chip, on which datasets are stored in the form of RFID tags and/or wherein the storage medium (81) consists of a label, on which datasets are stored in alphanumeric and/or barcode form.

11. The method according to any one of claims 6 to 10, wherein the suitability of the at least one material (Mⱼ) for processing (62) in the device (60) for product processing is checked during the ongoing production process.

12. The method according to any one of claims 6 to 11, wherein the application of the selection (210i) of the I measurement methods (S1, ..., SI) is at least ten times faster than the application (11) of the m measurement methods (S1, ..., Sm).

13. A system (500) configured for carrying out a method for identifying or distinguishing materials (Mⱼ) according to anyone of claims 1 to 12,
**characterized in that**
at least one local apparatus (510, 520, 530), in each case comprising at least one measuring device (400) for recording at least one actual signature (200j) each for materials (Mⱼ) and at least one local computer (541) communicatively connected to the at least one measuring device (400), and having a local database (4) for storing and/or processing the actual signature (220j) for storing and/or processing the actual signature (220j),
at least one central station (550), comprising a server (552) having a central database (7) for storing and/or processing the actual signatures (200j) of the at least one local apparatus (510, 520, 530); and
a network (560), which communicatively connects the at least one local computer (541) of the at least one local apparatus (510, 520, 530) and the server (552) of the central station (550).

14. The system (500) according to claim 13, wherein the at least one measuring device (400) of a local apparatus (510, 520, 530) comprises:
a plurality of measuring apparatuses (A1, ..., Au) for carrying out I different measurement methods (S1, ..., SI) on at least one sample (414) of a material (Mⱼ) for recording an actual signature (200j) of k time-dependent measurement values generated by v measuring apparatuses (A1, ..., Av) for carrying out m different measurement methods (S1, ..., Sm) for determining one enhanced target signature (100i) each from n measurement values for at least one target material (Mᵢ), wherein the numbers u, I and k are less than or equal to v, m and n, respectively, and
a data recording device (460), which is electrically connected to the u measuring apparatuses (A1, ..., Au) for synchronized recording of the k time-dependent measurement values of the actual signature (200j), and to at least one local computer (541).

15. The system (500) according to claim 13 or 14, wherein the system (500) comprises all measuring apparatuses (A1, ..., Av) for carrying out all m different measurement methods (S1, ..., Sm) for defining one enhanced target signature (100i) each from n measurement values for at least one target material (Mᵢ) such that the enhanced target signatures (100i) can be captured by the system (500) and can be stored in a non-volatile manner in the central database (7).

## Revendications

1. Procédé d'identification ou de discrimination de matériaux (Mⱼ) pour un processus de production, le procédé étant réalisé avec un système (500) et comprenant les étapes suivantes:
recueillir (610) une signature réelle (200j) sur un matériau (Mⱼ) à déterminer, par un utilisateur (511, 521, 531) d'un dispositif local (510, 520, 530) du système (500), dans lequel une signature réelle (200j) est un vecteur de k valeurs mesurées différentes obtenues en effectuant I différentes méthodes de mesure (S1, .... Sl) sur le matériau (Mⱼ) à déterminer;
demander (620) l'au moins un utilisateur (511, 521, 531) à un opérateur (551) d'une centrale (550) du système (500), la signature réelle (200j) étant transmise avec la demande;
mémoriser non volatile de la signature réelle (200j) transmise par l'au moins un utilisateur (511, 521, 531) sur un serveur (552) de la station centrale (550);
générer chacun une signature réelle dérivée (300j) en appliquant chacun une corrélation (220i) donnée par une matrice pour au moins un matériau cible (Mᵢ) à la signature réelle (200j), la corrélation (220i) étant obtenue par cette optimisation, en ce qu'un écart (δ) entre une signature cible étendue (100i) du matériau cible respectif (Mᵢ) et une signature cible dérivée (300i) du matériau cible respectif (Mᵢ) est minimisé, la signature cible étendue (100i) étant constituée de n valeurs de mesure différentes pour le matériau cible (Mᵢ), qui sont obtenues par m différents méthodes de mesure (S1, ..., Sm), et le nombre n est supérieur ou égal au nombre k et le nombre m est supérieur ou égal au nombre l, dans lequel la signature cible dérivée (300i) est obtenue en appliquant la corrélation (220i) à une signature cible (200i) pour le matériau cible respectif (Mᵢ), et dans lequel la signature cible (200i) pour le matériau cible respectif (Mᵢ) est un vecteur de k valeurs des mesure pour le matériau cible (Mᵢ) obtenues en effectuant les l différents méthodes de mesure du matériau cible (Mᵢ);
calculer d'une déviation (δij) de chacune des signatures réelles dérivées (300j) pour le matériau (Mⱼ) à partir de la signature cible dérivée (300i) du au moins un matériau cible (Mᵢ);
confirmer (630) à l'utilisateur qui sollicite (511, 521, 531) pour quels matériaux cibles (Mᵢ) l'écart respectif (δij) d'un matériau (Mⱼ) par rapport à un matériau cible (Mᵢ) est inférieur à une tolérance (Δ) définie pour le matériau cible (Mᵢ).

2. Procédé selon la revendication 1, les signatures réelles (200j) étant complétées par au moins un utilisateur (511, 521, 531) et/ou par l'opérateur (551) avec des métadonnées relatives à l'utilisateur (511, 521, 531) effectuant la demande et/ou à l'opérateur (551).

3. Procédé selon la revendication 2, dans lequel l'utilisateur (511, 521, 531) qui sollicite évalue l'identification ou la discrimination correcte du matériau (Mⱼ) sur confirmation (630) de l'opérateur (551) et rapporte le résultat de son évaluation (640) à l'opérateur (551) en tant que métadonnées.

4. Procédé selon l'une des revendications 2 à 3, dans lequel, pour chaque utilisateur (511, 521, 531) pour au moins un matériau cible (Mᵢ), la corrélation associée (220i) et la signature cible dérivée associée (300i) sont optimisées selon une combinaison (40) de critères d'optimisation (41a, ..., 41p) avec des pondérations associées (42a, ..., 42p), les pondérations (42a, ..., 42p) étant modifiées en fonction de métadonnées.

5. Procédé selon la revendication 3, dans lequel l'opérateur (551) et/ou un utilisateur (511, 521, 531) utilise les métadonnées et/ou les signatures réelles (200j) pour
étendre les signatures réelles (200j) par des valeurs de mesure enregistrées par des méthodes de mesure supplémentaires (A1, ..., Av) et les signatures cibles dérivées (300i) et nouvellement optimiser les corrélations (220i) pour au moins un matériau cible (Mᵢ), ou
créer l'article (Mⱼ) dans une base de données locale (4) et/ou dans la base de données centrale (7) comme nouvel article cible (Mᵢ).

6. Procédé selon la revendication 1, dans lequel, avant que la signature réelle (200j) ne soit recueillie (610), une sélection (210) de I méthodes de mesure parmi m méthodes de mesure différentes (S1, ..., Sm) est effectuée, où le nombre I est inférieur ou égal à m; mesurer (21) ensuite une signature réelle (200j) du matériau (Mⱼ) au moyen d'un dispositif de mesure (400) pour exécuter les I différentes méthodes de mesure (S1, .... SI); générer (22) ensuite une signature réelle dérivée respective (300j) à partir de la signature réelle mesurée (200j) du matériau (Mⱼ) en appliquant au moins une corrélation (220i) d'au moins un matériau cible (Mᵢ) associé chacun à un matériau cible (Mᵢ); puis en déterminant (23) un écart respectif (δij) des signatures réelles dérivées (300j) du matériau (Mⱼ) de la signature cible dérivée respective (300i) du au moins un matériau cible (Mᵢ); enfin, il y a une comparaison (24) de chaque écart (δij) avec une tolérance (Δ) spécifiée pour le matériau cible respectif (Mᵢ); et enfin une sortie (62) pour quels matériaux cibles (Mᵢ) l'écart respectif (δij) entre le matériau cible (Mᵢ) et le matériau (Mⱼ) est inférieur à la tolérance (Δ) du matériau cible respectif (Mᵢ).

7. Procédé selon la revendication 6, dans lequel une base de données cible (2) pour le stockage non volatile d'ensembles de données cibles comprenant les signatures cibles dérivées (300i), les sélections (210i) et les corrélations (220i) pour l'au moins un matériau cible (Mᵢ) est générée par les étapes suivantes:
appliquer (11) les m différentes méthodes de mesure (S1, .... Sm) pour générer une signature de cible étendue (100i) à partir d'au moins n valeurs mesurées pour au moins un matériau cible (Mᵢ);
créer (12) une base de données de mesure (1) de signatures de cibles étendues (100i);
optimer (14) avec un algorithme assisté par ordinateur d'une signature de consigne dérivée (300i) qui est générée par une corrélation associée (220i) à partir d'une signature cible associée (200i) qui est générée par une sélection (210i) des méthodes de mesure (S1, ..., Sm), l'optimisation (14) étant réalisée selon une combinaison (40) de critères d'optimisation (41a, ..., 41p) qui sont pourvus de pondérations associées (42a, ..., 42p) selon leur pertinence pour le traitement (62) d'un matériau (Mⱼ) dans un dispositif (60) pour un traitement de produit;
créer (15) de la base de données cible (2) à partir des signatures cibles dérivées (300i), sélections (210i) et corrélations (220i).

8. Procédé selon l'une des revendications 6 à 7, dans lequel dans une base de données (3) un sous-ensemble sélectionné d'enregistrements de données cibles, chacun consistant respectivement en la sélection (210i) des méthodes de mesure (S1,..., Sn), la signature cible (200i), la corrélation (220i) et/ou la signature cible dérivée (300i) d'au moins un matériau souhaité (Mᵢ), est mémorisée de manière non volatile, et/ou des enregistrements de données de test qui se composent des signatures réelles (200j), des signatures réelles dérivées (300j) et/ou des écarts (δij), étant enregistrés dans la base de données (3) pendant le traitement (62).

9. Procédé selon la revendication 8, dans lequel les enregistrements de données de test de la base de données (3) sont transmis automatiquement via un réseau numérique à une autre base de données externe (5) et dans lequel les enregistrements de données de test de la base de données (3) sont utilisés comme paramètres de commande pour un circuit de régulation pendant le traitement (62) dans un dispositif (60) pour le traitement du produit.

10. Procédé selon la revendication 9, dans lequel un support de mémorisation externe (81), sur lequel sont mémorisés des enregistrements de données cibles et/ou des enregistrements de données de test, est relié de manière lisible à un récipient, un emballage et/ou un lot d'un matériau cible (Mᵢ) ou un matériau (Mⱼ), et dans lequel le support de stockage externe (81) consiste en une puce d'identification par radiofréquence (RFID) sur laquelle sont stockés des enregistrements de données sous la forme d'étiquettes RFID et/ou dans lequel le support de stockage (81) consiste en une étiquette sur laquelle des enregistrements de données sont stockés sous forme alphanumérique et/ou sous forme de code barre.

11. Procédé selon l'une des revendications 6 à 10, dans lequel l'aptitude de l'au moins un matériau (Mⱼ) pour le traitement (62) dans le dispositif (60) pour le traitement du produit est vérifiée pendant le processus de production en cours.

12. Procédé selon l'une des revendications 6 à 11, dans lequel l'application de la sélection (210i) des l méthodes de mesure (S1, ..., Sl) est au moins dix fois plus rapide que l'application (11) des m méthodes de mesure (S1, ..., Sm).

13. Système (500) adapté pour effectuer un procédé d'identification ou de discrimination de matériaux (Mⱼ) selon l'une quelconque des revendications 1 à 12,
**caractérisé par le fait que**
au moins un dispositif local (510, 520, 530), comprenant chacun au moins un dispositif de mesure (400) pour recueillir au moins une signature réelle (200j) pour des matériaux (Mⱼ) et au moins un ordinateur local (541) connecté en communication avec le au moins un dispositif de mesure (400) et ayant une base de données locale (4) pour stocker et/ou traiter la signature réelle (200j),
au moins une station centrale (550) comprenant un serveur (552) avec une base de données centrale (7) pour stocker et/ou traiter les signatures réelles (200j) du au moins un dispositif local (510, 520, 530); et
un réseau (560) connectant de manière communicative le au moins un ordinateur local (541) du au moins un dispositif local (510, 520, 530) et le serveur (552) de la centrale (550).

14. Système (500) selon la revendication 13, ledit au moins un dispositif de mesure (400) d'un dispositif local (510, 520, 530) comprenant:
une pluralité d'appareils de mesure (A1, ..., Au) pour réaliser l différents méthodes de mesure (S1, ..., Sl) sur au moins un échantillon (414) d'un matériau (Mⱼ) pour recueillir une signature réelle (200j) à partir de k valeurs de mesure dépendantes du temps produites par v appareils de mesure (A1, ..., Av) pour réaliser m différents méthodes de mesure (S1, ..., Sm) pour déterminer respectivement une signature cible étendue (100i) à partir de n valeurs mesurées pour au moins un matériau cible (Mᵢ), les nombres u, I et k étant inférieurs ou égaux à v, m et n, respectivement ; et
un dispositif d'acquisition de données (460) relié électriquement aux u appareils de mesure (A1, ..., Au) pour l'acquisition synchronisée des k valeurs de mesure dépendantes du temps de la signature réelle (200j) et du au moins un ordinateur local (541).

15. Système (500) selon la revendication 13 ou 14, dans lequel le système (500) comprend tous les appareils de mesure (A1, ..., Av) pour exécuter tous les m méthodes de mesure différents (S1, ..., Sm) pour déterminer respectivement une signature cible étendue (100i) à partir de n valeurs mesurées pour au moins un matériau cible (Mᵢ), de sorte que les signatures cible étendue (100i) puissent être recueillis par le système (500) et mémorisées de manière non volatile dans la base de données centrale (7).
